(19) Europäisches Patentamt
European Patent Office
Office européen des brevets

(11) EP 2 863 875 B1

(12) EUROPEAN PATENT SPECIFICATION

(45) Date of publication and mention
of the grant of the patent:
**22.08.2018 Bulletin 2018/34**

(51) Int Cl.:
*C12Q 1/18* *(2006.01)*       *A61Q 11/00* *(2006.01)*
*A61K 8/41* *(2006.01)*       *A61K 8/06* *(2006.01)*
*A61Q 17/00* *(2006.01)*

(21) Application number: **13733206.0**

(22) Date of filing: **20.06.2013**

(86) International application number:
**PCT/US2013/046731**

(87) International publication number:
**WO 2013/192382 (27.12.2013 Gazette 2013/52)**

(54) **MOUTH RINSE EMULSIONS**

MUNDSPÜLEMULSIONEN

ÉMULSIONS DE BAIN DE BOUCHE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.06.2012 US 201213529064**

(43) Date of publication of application:
**29.04.2015 Bulletin 2015/18**

(73) Proprietor: **The Procter & Gamble Company
Cincinnati, OH 45202 (US)**

(72) Inventors:
• **SCOTT, Douglas, Craig**
**Loveland, Ohio 45140 (US)**
• **RAMJI, Niranjan**
**Mason, Ohio 45040 (US)**
• **TEPPER, Bruce, Ernest**
**Cincinnati, Ohio 45236 (US)**

(74) Representative: **Joos, Uta Susanne**
**Procter & Gamble Service GmbH**
**IP Department**
**Sulzbacher Strasse 40 - 50**
**65824 Schwalbach am Taunus (DE)**

(56) References cited:
**WO-A1-96/15770        WO-A1-2012/076295
WO-A1-2012/076309      WO-A2-2008/005705**

**Description**

TECHNICAL FIELD

The present invention relates to mouth rinse compositions containing one or more oral care actives and flavor oils formulated in the form of oil-in-water emulsions.

BACKGROUND OF THE INVENTION

Oral care products such as mouth rinses are routinely used by consumers as part of their oral care hygiene regimens to provide therapeutic, hygiene and cosmetic benefits. Therapeutic benefits include caries prevention which is typically delivered through the use of various fluoride salts; gingivitis and periodontal disease prevention by the use of antimicrobial agents such as triclosan, stannous fluoride, quaternary ammonium compounds or essential oils; or hypersensitivity control through the use of ingredients such as strontium chloride, stannous fluoride or potassium nitrate. Hygiene and cosmetic benefits include the control of plaque and calculus formation, removal and prevention of tooth stain, tooth whitening, breath freshening, and overall improvements in mouth feel impression which can be broadly characterized as mouth feel aesthetics. Calculus and plaque along with behavioral and environmental factors lead to formation of dental stains, significantly affecting the aesthetic appearance of teeth. Behavioral and environmental factors that contribute to tooth staining include regular use of coffee, tea, cola or tobacco products, and also the use of certain oral products containing ingredients that promote staining, such as cationic antimicrobial agents.

Mouth rinses are thus formulated to contain one or more oral care agents to address the above needs in a liquid carrier typically containing one or more of water as the main solvent component, other solvent(s) such as ethanol, surfactant(s), humectants(s) flavoring agent(s) and sweetening agent (s). In addition to product safety considerations, formulating mouth rinse products requires careful balancing of many factors including: (1) chemical stability, compatibility and bioavailability of the active components to deliver the intended therapeutic and/or cosmetic benefits; (2) taste and mouthfeel characteristics of the product for consumer acceptability and also to encourage user compliance with repeated use and longer retention in the mouth for efficacy; (3) avoiding or mitigating negatives during use such as staining that may be derived from cationic antimicrobial components; and (4)physical stability of the product for acceptable shelf life and commercial viability.

Mouth rinses in the market are typically "clear" or transparent products, i.e., homogeneous or single-phase products wherein all components are completely solubilized in the liquid carrier being water or water/solvent mixtures. Products that are clear and homogeneous in appearance have generally been thought to be aesthetically pleasing and preferred by consumers. For example, WO 2012/076309 A1 discloses antimicrobial compositions in micro-emulsion format that are capable of forming turbid suspension on dilution with water and WO 2012/076295 A1 discloses mouthwash compositions that are substantially free of C1 to C3 alcohol. WO 2008/005705 A1 discloses metal-containing materials as well as formulations and uses thereof. In addition, non-homogeneous or phase-separated products may result in inconsistent delivery of actives during use. Mouth rinses may contain one or more substantially water-insoluble components such as flavoring agents or flavorants for imparting a pleasant taste. Examples of flavorants are flavor oils such as peppermint, spearmint, wintergreen and cinnamon. Flavor oils belong to the class of materials called "volatile oils", which can vary in water solubility but are generally not readily soluble in an aqueous system at concentrations to provide desired flavor effects or impact. Therefore, to create clear solutions, solubilization agents are required. Such solubilization agents include solvents such as ethanol, propylene glycol, or polyethylene glycol and surfactants such as poloxamers and polysorbates. Solvents are not always desirable because they can impart an unpleasant taste or sensation - specifically, a chemical taste, bitterness or burning. Solvents can also be expensive and are not ideal for handling in processing plants in large quantities. For example, ethanol is flammable. Surfactants used at high levels can also impart a bitter or soapy taste as well as having the potential for causing tissue irritation and oral cavity desquamation. Additionally, surfactants can have negative effects on the bioavailability of some active ingredients. For example, surfactants can decrease the bioavailability of cationic antimicrobials such as cetyl pyridinium chloride (CPC) and chlorhexidine by forming mixed micelles in an aqueous vehicle, these micelles affecting bioavailability. Furthermore, the bioavailability of CPC can be reduced by the flavor oils themselves and by addition of electrolytes or other water soluble components such as fluoride and saccharin. In the case of flavor oils, high levels and the use of the relatively more water-insoluble flavors, such as peppermint and spearmint, are very difficult to formulate as clear solutions with high CPC bioavailability. Clear CPC formulas are generally restricted to the use of more water soluble flavors such as wintergreen and cinnamon at modest levels typically no more than about 0.15%.

Although satisfactory in many respects, a need remains for further improvements in formulating aqueous mouth rinses, specifically containing much higher levels than commonly used of essentially water-insoluble components such as flavor oils. Flavor oils are key components of mouth rinses because of their taste and antimicrobial benefits The present invention addresses the difficulties associated with formulating high levels of flavor oils in combination with other actives via use of emulsions, specifically oil-in-water emulsions

SUMMARY OF THE INVENTION

The present invention is directed to oral care mouth rinse compositions, formulated as stable oil-in-water emulsions comprising:

(a) from 0.025 % to 0.1 % by weight of cetylpyridinium chloride as antimicrobial agent,
(b) at least 0.05 % by weight of an essentially water-insoluble volatile oil, and
(c) an orally-acceptable carrier comprising water at a level of at least 50 % by weight of the composition, and,
(d) less than 0.1% total additional surfactant by weight of the composition, wherein the composition is a stable oil-in-water nano-emulsion having a dispersed phase comprising oil droplets having an average mean particle size of 30 to 350 nm or less, wherein the nano-emulsion does not form spontaneously, but requires energy input for formation, and are not thermodynamically stable once formed.

Examples of quaternary ammonium antimicrobial agent include cetylpyridinium chloride (CPC), tetradecylpyridinium chloride, N-tetradecyl-4-ethyl pyridinium chloride or domiphen bromide.
These and other features, aspects, and advantages of the present invention will become evident to those skilled in the art from the detailed description which follows.

DETAILED DESCRIPTION OF THE INVENTION

While the specification concludes with claims particularly pointing out and distinctly claiming the invention, it is believed that the present invention will be better understood from the following description.
All percentages and ratios used hereinafter are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated. All measurements referred to herein are made at about 25°C unless otherwise specified.
Herein, "comprising" means that other steps and other components which do not affect the end result can be added. This term encompasses the terms "consisting of" and "consisting essentially of."
As used herein, the word "include," and its variants, are intended to be non-limiting, such that recitation of items in a list is not to the exclusion of other like items that may also be useful in the materials, compositions, devices, and methods of this invention.
As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.
By "oral care composition" is meant a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral care composition may be in various forms including toothpaste, dentifrice, tooth gel, subgingival gel, mouth rinse, mousse, foam, denture product, mouthspray, lozenge, chewable tablet or chewing gum. The oral care composition may also be incorporated onto strips or films for direct application or attachment to oral surfaces.
The term "mouth rinse", as used herein, includes liquid formulations referred in the art as mouthwashes or dental rinses, mouth sprays, dental solutions and irrigation fluids.
The term "dentifrice", as used herein, means paste, gel, or liquid formulations unless otherwise specified. The dentifrice composition may be a single phase composition or may be a combination of two or more separate dentifrice compositions. The dentifrice composition may be in any desired form, such as deep striped, surface striped, multilayered, having the gel surrounding the paste, or any combination thereof. Each dentifrice composition in a dentifrice comprising two or more separate dentifrice compositions may be contained in a physically separated compartment of a dispenser and dispensed side-by-side.
The term "dispenser", as used herein, means any pump, tube, or container suitable for dispensing compositions such as dentifrices.
The term "teeth" refers to natural teeth as well as artificial teeth or dental prosthesis.
The terms "pharmaceutically acceptable carrier", "orally acceptable carrier" or "excipients" include safe and effective materials and conventional additives such as those used in oral care compositions including but not limited to fluoride ion sources, antimicrobial agents, anti-inflammatory agents, anti-calculus or anti-tartar agents, desensitizing agents, peroxide sources, abrasives such as silica, buffering agents, alkali metal bicarbonate salts, thickening materials, humectants, water, surfactants, emulsifying agents, anti-stain agents, tooth substantive agents, titanium dioxide, xylitol, essential oils, a coolant, a sweetening agents or other sensates and coloring agents.

The term "essential oils" as used herein refers to "volatile oils" distilled or expressed from plants and constituents of these volatile oils. Typical essential oils and their main constituents are those obtained for example from thyme (thymol, carvacrol), oregano (carvacrol, terpenes), lemon (limonene, terpinene, phellandrene, pinene, citral), lemongrass (citral, methylheptenone, citronellal, geraniol), orange flower (linalool, $\beta$-pinene, limonene), orange (limonene, citral), anise (anethole, safrol), clove (eugenol, eugenyl acetate, caryophyllene), rose (geraniol, citronellol), rosemary (borneol, bornyl esters, camphor), geranium (geraniol, citronellol, linalool), lavender (linalyl acetate, linalool), citronella (geraniol, citronellol, citronellal, camphene), eucalyptus (eucalyptol); peppermint (menthol, menthyl esters), spearmint (carvone, limonene, pinene); wintergreen (methyl salicylate), camphor (safrole, acetaldehyde, camphor), bay (eugenol, myrcene, chavicol), cinnamon (cinnamaldehyde, cinnamyl acetate, eugenol), tea tree (terpinen-4-ol, cineole), and cedar leaf (a-thujone, $\beta$-thujone, fenchone). Essential oils are widely used in perfumery and as flavorings, medicine and solvents [See Kirk-Othmer Encyclopedia of Chemical Technology, 4th Edition and The Merck Index, 13th Edition].

By "essentially water-insoluble" herein in reference to flavor oils (also referred to as volatile oils or essential oils) and other solutes, is meant that the flavor oil or solute has a solubility in water of no more than 0.1% at about 25°C.

Active and other ingredients useful herein may be categorized or described by their cosmetic and/or therapeutic benefit or their postulated mode of action or function. However, the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed.

The term "emulsion" as used herein means a suspension or dispersion of tiny "droplets" of a first liquid (dispersed or internal phase) in a second liquid (continuous or external phase), wherein the first liquid and the second liquid are normally immiscible (un-blendable). Emulsions are part of a more general class of two-phase systems of matter called colloids. Although the terms "colloid" and "emulsion" are sometimes used interchangeably, "emulsion" is used when both the dispersed and the continuous phases are liquid. Types of emulsions include (1) oil-in-water, where an oil is the dispersed phase while water is the continuous phase, (2) water- in-oil, wherein water is the dispersed phase while oil is the continuous phase, and (3) multiple emulsions such as oil-water-oil. Whether an emulsion turns into a water-in-oil emulsion or an oil-in-water emulsion depends on the volume fraction of both phases and on the type of emulsifier or surfactant. Typically, emulsions do not form spontaneously and are thermodynamically unstable. Energy input through shaking, stirring, homogenizing, or exposure to power ultrasound is needed to form an emulsion. Over time, emulsions tend to revert to the stable state of the individual phases comprising the emulsion.

The terms "micellar solution" and "micro-emulsion" are synonyms and are colloidal systems that can form spontaneously by "solubilizing" oil molecules with a mixture of surfactants, co-surfactants and solvents. Micro-emulsions do not require energy input for formation, and are thermodynamically stable once formed. The size of a "droplet" in a micellar solution or micro-emulsion is on the order of ≤10 nm.

The term "nano-emulsion" as used herein means a colloidal system that does not form spontaneously; requires energy input for formation and is not thermodynamically stable. The droplet size for a nano-emulsion is from about 10 to about 500 nm.

The terms "macro-emulsion" or "coarse emulsion" are synonyms and are colloidal systems that cannot form spontaneously. They require energy input for formation, and are not thermodynamically stable once formed. The size of a "droplet" in a macro-emulsion would be >500 nm.

In one embodiment of the present invention, mouth rinse compositions formulated as oil-in-water emulsions are provided comprising at least about 0.025% of a cationic antimicrobial agent comprising one or a mixture of quaternary ammonium compounds such as cetylpyridinium chloride, cetyl pyridinium fluoride, tetradecylpyridinium chloride, N-tetradecyl-4-ethyl pyridinium chloride, domiphen bromide, benzalkonium chloride or benzethonium chloride and at least about 0.1% of an essentially water-insoluble flavor oil, wherein the mean particle size of the oil droplets in the emulsion are about 350 nm or less and the ratio of oil to water is from about 0.05:99.5 to about 5:95. The present emulsions are "true" emulsions as defined herein, i.e., being two phase systems and not homogeneous, rather than being micellar solutions, which are formed by solubilizing oil molecules with a mixture of surfactants and/or solvents. To prepare the present emulsions, some form of mechanical energy input is used to create oil droplets in the desired mean size range of from about 30 to about 350 nm, preferably 30 to 200 nm. The present emulsions do not require the surfactant concentrations typically needed to form micellar solutions or micro-emulsions. Because of many undesirable side-effects caused by surfactants, this is disadvantageous or prohibitive for many applications.

The cationic antimicrobial agents used herein effectively promote oral hygiene and health, particularly by controlling plaque and calculus proliferation and also function as surfactant or emulsifying agent. Formulating the mouth rinses as emulsions as opposed to clear solutions is advantageous because it negates many of the limitations of clear rinses and provides other advantages as follows:

- No need for high levels of additional surfactant thereby avoiding negative effects of surfactant such as on bioavailability of the cationic quaternary ammonium antimicrobial agent
- No need for high levels of alcohol or other solvent, thereby reducing cost and avoiding negatives from such solvents, including safety and taste concerns,
- Ability to flavor with a wider range of flavor oils including those that are more hydrophobic and less water soluble such as peppermint and spearmint,
- Ability to use higher than currently practicable levels of flavor oils for flavor impact or extra benefits without using high levels of alcohol or other solvent,
- Ability to provide clear as well as cloudy/translucent appearance, of which the latter can be a visual signal of being different and/or efficacious,
- Provision of a different mouth feel rinse experience,
- Provision to enhance deposition of antimicrobial active(s) for improved efficacy, and
- Ability to include electrolytes such as fluoride, nitrate, phosphate, pyrophosphate and other salts which would normally have a negative impact on the bioavailability of the cationic quaternary ammonium antimicrobial agent and/or stability of the mouth rinse.

Formulation and processing of emulsions is an important aspect of emulsion stabilization. Reduction of surface tension at the oil-water interface is needed to stabilize emulsions which are thermodynamically unstable compared to micellar solutions of oils of which many are "clear" rinses. Additionally, some form of mechanical energy input is often needed to create emulsion droplets of small particle sizes, particularly < 500 nm. A surface active agent or surfactant would concentrate at oil-water interfaces, thereby reducing surface tension which is desirable to enable dispersion of the oil phase and creation of small oil droplets to form an oil-in-water emulsion. Mechanical energy input via typical emulsion processing equipment (such as a rotor-stator mixer device or a homogenizer) can also break up the oil phase to produce smaller droplets. The surfactant then aids in stabilization and maintaining the small particle size. Even with addition of mechanical energy, emulsions are not thermodynamically stable and phases will separate over time. Emulsions are only "kinetically" stable and this stability can vary from minutes to years.

Chemical energy effects can also be leveraged to enhance emulsion formation thus minimizing the level of energy input to produce small oil droplets. For example, 1) selection of surfactants and other emulsifiers for optimal surface tension reduction, 2) using optimal solvents for solubilization of oil and water phase ingredients prior to combination of phases, 3) phase dilution procedure, and 4) order of addition. Optimization of the formulation and the method of making can result in emulsions of very small droplet size with relatively little energy input.

To produce a formula for practical commercial use as mouth rinse, it is ideal to have at least a 1-2 years shelf life or stability. By stability herein is meant that the emulsion is stable against phase separation under storage conditions up to about 40 - 50 °C, freeze-thaw cycling and vibrational forces such as encountered during shipping. This is particularly difficult in the case of a mouth rinse which has low viscosity, i.e., the viscosity of the external aqueous phase. The viscosity of the aqueous phase in the case of an oil-in-water emulsion, affects the kinetic stability of the emulsion. Typically this would be approached by thickening and structuring of the external phase in order to slow oil droplet movement and reduce collisions, which can result in coalescence of droplets and formation of fewer but larger droplets. External phase thickening/structuring of mouth rinses is generally undesirable because desired viscosities for oral cavity rinsing are generally about 1 - 5 cps. Therefore, it is particularly important and challenging to maintain small droplet size in the absence of external thickening and structuring.

The nature of the oil phase has a significant effect on the ability to formulate emulsions which are kinetically stable long term. Common emulsions, such as hand and body lotions or creams and food emulsions such as mayonnaise and salad dressings, typically use "fixed oils", which include materials such as mineral oil, petrolatum, and vegetable oils. Fixed oils are relatively easier to formulate and stabilize due to the fact that they have essentially no water solubility, which is advantageous for oil-in-water emulsions where the continuous or external phase is aqueous. Conversely, volatile oils, while generally considered water insoluble, have some level of water solubility. This slight water solubility makes formulating and stabilization of emulsions containing volatile oils, such as flavor oils difficult. This is primarily due to the effect called Ostwald Ripening, which is an energetically driven phenomenon and known to occur in oil-in-water emulsions when the oil molecules have appreciable solubility in water. Ostwald Ripening occurs without oil droplet collision and coalescence. It occurs because flavor oils molecules can diffuse out of the oil droplet phase into the external water phase and then diffuse into larger droplets. It is a spontaneous and thermodynamically unavoidable phenomenon because larger particles are energetically favored.

The combination of destabilization by droplet collisions and coalescence, in addition to Ostwald Ripening in the case of volatile oils, can lead to the oil phase eventually becoming one big droplet to minimize the total surface area (lowest surface energy). When this happens, the emulsion becomes two separate phases. Depending on the composition and processing, this may take anywhere from minutes to tens of years. These formulation challenges have been solved by the present invention, which provides a shelf stable oil-in-water emulsion in a low viscosity and unstructured external phase, using relatively high levels of volatile or flavor oils.

Emulsion Stability Demonstration

To demonstrate the importance of particle size as related to stability, an experiment was conducted by processing a prototype CPC + flavor oil formula with different energy input. Lower energy processing was achieved using a typical rotor-stator device (IKA T25 UltraTorrex). Various energy input times and speeds were assessed. High energy input was achieved using a specialized homogenizer (Microfluidics Microfluidizer). Various pressures were assessed.

A range of particle sizes was achieved depending on processing conditions - mean particle size ranged from ~ 100 - 8000 nm. Particle size measurements were conducted using the the Zetasizer Nano described below. All samples were exposed to 3 cycles of freeze-thaw to assess physical stability. Freeze thaw is a standard accelerated condition to assess emulsion stability. Finished emulsions of various oil droplet size were placed in a freezer at ~ -18 °C and allowed to freeze for 2 days. Samples were then removed and allowed to thaw at room temperature. This procedure was repeated for two more cycles and then assessed for particle size and visual examination. The table below summarizes the pre and post mean particle size as well a visual assessment of the samples.

| Process Condition | Initial Particle Size (nm) | Post Freeze-Thaw Particle Size (nm) | Post Freeze-Thaw Visual Assessment |
|---|---|---|---|
| IKA Low RPM, 1 min | 7450 | 6968 | Phase Separation - Delineation Noticeable |
| IKA Med RPM, 1 min | 5850 | 8048 | Phase Separation - Delineation Noticeable |
| IKA High RPM, 1 min | 2144 | 4793 | Phase Separation - Delineation Noticeable |
| IKA Low RPM, 5 min | 7592 | 6655 | Phase Separation - Delineation Noticeable |
| IKA Med RPM, 5 min | 828 | 2323 | Phase Separation - Delineation Noticeable |
| IKA High RPM, 5 min | 489 | 478 | Phase Separation - Delineation Noticeable |
| MicroFluidizer 1000 PSI | 245 | 286 | Slight Reversed Creaming |
| MicroFluidizer 7000 PSI | 156 | 200 | Slight Reversed Creaming |
| MicroFluidizer 13,000 PSI | 131 | 155 | Slight reversed Creaming |
| MicroFluidizer 29,500 PSI | 115 | 122 | Slight reversed Creaming |

Creaming (or reversed creaming) is associated with rising or settling of oil droplets based on droplet density and does not involve actual coalescence, significant change in particle size mean, or actual phase separation (noticeable oil phase separation). Creaming is reversible with minor agitation. Phase separation indicates significant coalescence and actual separation of oil. As the data show, at a starting particle size of ~ 250 nm or less, little change in mean particle size is observed and only reversed creaming results. The very large starting sizes ~7000 - 8000 nm were not well emulsified to begin with so they were already very unstable. Sizes in the range of 800 - 6000 showed a large increase in mean particle size with freeze thaw.

Particle Size and Distribution Comparison

The following table compares prototype CPC emulsions vs. common low viscosity emulsions containing low levels of volatile oils, specifically beverages. Some beverages are "clear" and use water soluble extracts as flavorants; others use essentially water insoluble flavors and are emulsions - also called "clouds". Beverage emulsions typically

have $\geq 0.5$ um mean droplet size. They are also not as physically stable over time with regard to emulsification. This is acceptable for the beverage industry because beverages do not require long term stability, as compared to a drug/drug-like product, because the turnover of beverages in distribution channels is rapid.

As shown below, the present CPC emulsion prototypes have a significantly smaller particle size (Z) and a much narrower particle size distribution (reported as polydispersity index, PDI)

| Product | Z-Ave D (nm) | PDI |
| --- | --- | --- |
| Emulsion Mouth rinse #1 (0.1% CPC + 0.3% oil) | 188.63 | 0.082 |
| Emulsion Mouth rinse #2 (0.1% CPC + 0.3% oil) | 120.03 | 0.04 |
| Gatorade | 568.33 | 0.20 |
| Diet Orange Crush | 519.17 | 0.34 |
| Diet Mountain Dew | 620.1 | 0.36 |
| Vitamin Water | 715.7 | 0.24 |

Particle size measurements were performed using the Zetasizer Nano which uses a process called Dynamic Light Scattering (DSL). Dynamic light scattering (also known as PCS-Photon Correlation Spectroscopy) measures Brownian motion and relates this to the size of the particle. This is done by illuminating the particle with a laser and analyzing the intensity fluctuations in the scattered light. If a small particle is illuminated by a light source such as a laser, the particle will scatter the light in all directions. If a screen is held close to the particle, the screen will be illuminated by the scattered light. When the single particle is replaced by thousands of stationary particles the screen would show a speckle pattern. The speckle pattern will consist of areas of bright light and dark areas. An important feature of Brownian motion for DSL is that small particles move quickly and large particles move slowly. The relationship between the size of the particle and the speed due to Brownian motion is defined in the Stokes - Einstein equation. As the particles are constantly in motion the speckle pattern will also appear to move. As particles are constantly in motion the constructive and destructive phase addition of light scattering will cause the bright and dark areas to grow and diminish in intensity- or put in another way the intensities appear to fluctuate. The Zetasizer Nano system measures the rate of the intensity fluctuations and then uses this to calculate the size of the particles using mathematical algorithms.

Peak statistics are calculated using the expressions given below where $Y_i$ is the Y value of the $i^{th}$ Y axis class/bin and $X_i$ is the X axis value in the center of the X axis class/bin. The Y axis here is the Intensity (%) while the X axis is the diameter (nm). Area is defined as the area under each peak, relative to the total area of the distribution. Mean is defined as the average value of the peak, weighted by the Y axis parameter.

$$\% \text{ Area} = \sum_i Y_i$$

$$\text{Mean} = \sum S(i)I(i) / \text{ Area}$$

$$\text{Polydispersity or Width of the Peak} = \text{Square root} \left( \left( \sum_{Xi2Yi} / \% \text{ Area} \right) - \text{Mean}^2 \right)$$

Polydispersity Index (PDI) is a number calculated from a simple 2 parameter fit to the correlation data (the cumulants analysis). The Polydispersity Index is dimensionless and scaled such that values smaller than 0.05 are seen with highly monodisperse standards. Values greater than 0.7 indicate that the sample has a very broad size distribution and is probably not suitable for the dynamic light scattering (DLS) technique. The various size distribution algorithms work with data that fall between these two extremes. The calculations for these parameters are defined in the ISO standard document 13321:1996 E and ISO 22412:2008.

Particle Size Effects on Visual Appearance of Emulsions

In addition to affecting stability of emulsions, particle size also affects its visual appearance. Particle sizes of -200 nm or less have little effect on the manner in which light passes through a product, resulting in the emulsion appearing clear or translucent. Emulsions with oil droplet sizes below -200 nm can appear translucent because light can penetrate through the emulsion without being scattered. Particles sizes of -250 nm and greater would appear opaque due to scattering of light. Polydispersity (or particle size distribution) is important because even if the mean particle size is less than 200 nm (150 nm, for example), there may be larger particles in the distribution, i.e. greater than 200 nm, or greater than 250 nm, resulting in the product appearing opaque. If it is desired for the emulsion to not appear opaque or cloudy, the largest particles in the distribution must not be ~> 250 nm. The product can appear opaque/cloudy even if only a small fraction of particles exceeds the size limit for light scattering. Thus, emulsions provide the ability to create different final appearance of a product for different applications, ranging from clear to varying degrees of translucency and opacity via manipulation of droplet particle sizes. This is important in designing a distinct and visually aesthetically pleasing product.

Technical Performance Evaluation of Emulsion Mouth Rinse

Emulsions can offer better performance compared to their clear counterparts. This is believed due to: 1) emulsions being better delivery systems based on destabilization when interacting with the oral surfaces and biofilms due to the fact that emulsions are thermodynamically unstable systems (vs. thermodynamically stable micellar solutions), and 2) the high oil load offered by the emulsions provide greater biofilm dispersion and/or antimicrobial effects resulting in better performance and potentially better biofilm penetration of other antimicrobial ingredients such as CPC. The enhanced efficacy of the present emulsion mouth rinses is demonstrated in the following tests/

Two marketed rinses for gum health are Listerine (Johnson & Johnson) and Crest Pro Health (Procter & Gamble). Both are clear rinses, not emulsions as defined herein. The active antimicrobial ingredients in the Listerine product include a four-component mixture of the volatile oils - thymol, eucalyptol, menthol and methyl salicylate at a total level of ~ 0.27%. Additional flavor oils are also added. The oils are solubilized using a high level of ethanol (about 20%) and surfactant. The active ingredient in Crest Pro Health is 0.07% CPC. The formula also contains ~ 0.12% flavor oils, a low level of nonionic surfactant and no alcohol. Both products provide similar performance when tested clinically.

In a 6-month Plaque Clinical Trial, Listerine and Crest Pro Health rinse were shown to be statistically equivalent, providing 26.8% and 31.1% plaque reduction vs. baseline, respectively. This study was a randomized, double-blind, parallel groups, single-center study. Seventy-eight healthy adults were enrolled in the study. Treatments included Listerine Cool Mint (essential oils + alcohol), and Crest Pro Health rinse (700 ppm CPC, 0% alcohol). Four weeks before the baseline visit, subjects received a prophylaxis and were instructed to brush twice daily in a manner to approach optimum gingival health. At the end of the 4-week period, subjects were assigned to treatment and instructed to use 20 ml of their assigned product for 30 seconds after brushing twice daily during a 21-day treatment phase. Plaque removal by brushing was prevented during the treatment phase for one mandibular quadrant (experimental gingivitis region) by means of a specially manufactured tooth shield. Efficacy measurements were obtained at baseline and at the end-of-treatment including the Modified Quigley-Hein Plaque Index (Am J. Dent. 2005; 18: 15A-17A).

The clinical plaque reduction performance of prototype emulsions containing 1000 ppm CPC and a high loading of flavor oils were evaluated in a 6-week and 12-week trial compared to a marketed product Cool Mint Listerine.

The 6-Week study was a randomized, controlled, examiner-blinded, 3-treatment, parallel study with measures including plaque regrowth. Treatments included: 1) a prototype emulsion mouth rinse containing 1000 ppm CPC and 0.3% flavor oils, 2) Cool Mint Listerine, and water (brushing only). The study included ~ 150 subjects which were assigned to treatments and instructed to use 20 ml of their assigned product for 30 seconds after brushing twice daily during the 6 week period. Plaque was assessed using the Rustogi Modification of the Navy Plaque Index. Efficacy measurements were obtained at baseline and at the end-of-treatment. Subjects were not given a prophylaxis prior to the study.

The 12-Week study was a 3-month, randomized, controlled, Examiner-blinded, 3-treatment, parallel study with measures including plaque regrowth. Treatments included: 1) a prototype emulsion mouth rinse containing 1000 ppm CPC and 0.3% flavor oils, 2) Listerine Cool Mint, and water (brushing only). The study included ~ 150 subjects which were assigned to treatments and instructed to use 20 ml of their assigned product for 30 seconds after brushing twice daily during the 3 month treatment period. Plaque was assessed using the Turesky modification of the Quigley-Hein Plaque Index. Efficacy measurements were obtained at baseline and at the end-of-treatment. Subjects were not given a prophylaxis prior to the study.

In both studies, the emulsion prototype was statistically and meaningfully better than Listerine. Emulsion A contained 1000 ppm CPC + 0.3 % flavor oils (peppermint + other essential oils); Emulsion B contained 1000 ppm CPC + 0.3% flavor oils (peppermint + other essential oils). The other essential oils in the two emulsions had different components. Emulsion A provided 41% plaque reduction vs. baseline after 6 weeks compared to 31% plaque reduction for the Listerine rinse. Emulsion B provided 48% plaque reduction vs. baseline after 12 weeks compared to 29% for the Listerine rinse.

Another study to evaluate the anti-microbial effects of the present mouth rinse emulsions with high flavor oil loading compared to traditional clear rinses is described in Example 2 below.

Cationic Antimicrobial Agents

Cationic antimicrobial agents included in the present compositions include quaternary ammonium salts which provide effectiveness in killing, and/or altering metabolism, and/or suppressing the growth of, microorganisms which cause topically-treatable infections and diseases of the oral cavity, such as plaque, caries, gingivitis, and periodontal disease. The level of antimicrobial agent is dependent on the chemical nature of the agent and other factors and may comprise from about 0.01% to about 5.0%, by weight of the composition.

The quaternary ammonium compounds in the compositions of the present invention include those in which one or two of the substituents on the quaternary nitrogen has a carbon chain length (typically alkyl group) from about 8 to about 20, typically from about 10 to about 18 carbon atoms while the remaining substitutents (typically alkyl or benzyl group) have a lower number of carbon atoms, such as from about 1 to about 7 carbon atoms, typically methyl or ethyl groups. Cetylpyridinium chloride, cetyl pyridinium fluoride, tetradecylpyridinium chloride, N-tetradecyl-4-ethyl pyridinium chloride, domiphen bromide, benzalkonium chloride, benzethonium chloride, methyl benzethonium chloride, dodecyl trimethyl ammonium bromide, dodecyl dimethyl (2-phenoxyethyl) ammonium bromide, benzyl dimethoxystearyl ammonium chloride, quaternized 5-amino-1,3-bis(2-ethyl-hexyl)-5-methyl hexa hydropyrimidine, lauryl trimethylammonium chloride, cocoalkyl trimethylammonium chloride, cetyl trimethylammonium bromide, di-isobutylphenoxyethyl-dimethylbenzylammonium chloride, dodecyl trimethyl ammonium bromide, are exemplary of typical quaternary ammonium antimicrobial agents, are exemplary of typical quaternary ammonium antimicrobial agents. Other compounds are bis[4-(R-amino)-1-pyridinium] alkanes as disclosed in U.S. No. 4,206,215 to Bailey. The pyridinium compounds are the preferred quaternary ammonium compounds, particularly preferred being cetylpyridinium, or tetradecylpyridinium halide salts (i.e., chloride, bromide, fluoride and iodide). Particularly preferred is cetylpyridinium chloride. The quaternary ammonium antimicrobial agents are included in the present invention at levels of at least about 0.025% , at least about 0.035%, at least about 0.045%, at least about 0.05% or at least about 0.07% by weight of the composition, depending on the specific application. For applications intended to provide high antimicrobial efficacy, the level of quaternary ammonium agent will typically range from about 0.025% to about 0.1%, taking into consideration factors such as bioavailability as well as the tooth staining that may be caused by these agents.

Bioavailability of the quaternary ammonium agent such as CPC in rinse formulations is measured using the *in vitro* Disk Retention Assay (DRA). The DRA method is described in commonly assigned application WO 05/072693 and in S. J. Hunter-Rinderle, et al., "Evaluation of Cetylpyridinium Chloride-Containing Mouthwashes Using In Vitro Disk Retention and Ex Vivo Plaque Glycolysis Methods," J. Clin. Den., 1997, 8:107-113. These assays are recommended for use in the proposed OTC monograph (Federal Register Vol. 68, No. 103 Part 356, "Oral Health Care Drug Products For Over-The-Counter Human Use; Antigingivitis/Antiplaque Drug Products; Establishment of a Monograph: Proposed Rules "). This method is designed as a performance assay to analyze mouth rinse formulations containing from about 0.03% to about 0.1% CPC to quantitatively determine the "free" ("unbound") or "bioavailable" level of CPC needed for clinical efficacy. The DRA measures the amount of CPC "binding" to standardized cellulose filter disks during filtration of an undiluted mouth rinse sample. The "bioavailable" CPC binds to the hydroxyl groups on the cellulose fiber during filtration while CPC, which has been rendered "non-bioavailable" (or "bound")" through interactions with mouth rinse components, simply passes through the filter paper, i.e., the positive charge on the compound is no longer available for binding to the negatively charged cellulose disks. In this way, the DRA test provides an estimate of the amount of CPC available for activity, i.e., binding to bacteria and mucosal surfaces, during use of the mouth rinse. DRA measurements of CPC availability have been positively correlated to results of *in vitro* microbiological assays and *in vivo* germ kill tests. Historically, cellulose fibers have been used in other applications to similarly monitor biological activity of drug actives ("Dairy Products" in Official Methods of Analysis of the Association of Chemical Analytical Chemists. 13th ed., 1980, Chapter 16:256). The method has been validated and shown to perform with acceptable accuracy, precision, and selectivity.

Mouth rinse formulations comprising from about 0.035 to about 0.1% CPC would pass the DRA test if assay results show the level of bioavailable CPC to be ≥ 324 ppm. For example, a formulation comprising 0.05% CPC at 72% bioavailability would provide 360 ppm CPC. Testing of products containing bioavailable levels of CPC of ≥ 324 ppm demonstrates positive clinical (antigingivitis, antiplaque) outcomes. Determination of CPC bioavailability in a finished product is important to product performance as it readily defines the amount (concentration) of active available for deposition at the site of action. Because the positively charged (cationic) hydrophilic region is critical to antimicrobial activity, any formulation component that diminishes the activity of this cationic group or that competes with the group may inactivate the product. Desirably, a formulation containing 0.05% CPC would have at least about 65% bioavailability to deliver at least about 324 ppm bioavailable CPC. A formulation containing a lower level of CPC such as 0.04% would need to have at least about 81% bioavailability to deliver the minimum required level of bioavailable CPC for antigingivitis efficacy. Depending upon the particular application and the concentration of CPC or other quaternary ammonium agent, about 50% bioavailabilty may be acceptable.

Additional Antimicrobial Agents

The present compositions may comprise additional cationic antimicrobials such as metal ion sources that provide stannous ions, zinc ions, copper ions, or mixtures thereof. The metal ion source can be a soluble or a sparingly soluble compound of stannous, zinc, or copper with inorganic or organic counter ions. Examples include the fluoride, chloride, chlorofluoride, acetate, hexafluorozirconate, sulfate, tartrate, gluconate, citrate, malate, glycinate, pyrophosphate, metaphosphate, oxalate, phosphate, carbonate salts and oxides of stannous, zinc, and copper.

Stannous, zinc and copper ions have been found to help in the reduction of gingivitis, plaque, sensitivity, and improved breath benefits. The composition may comprise from about 50 ppm to about 20,000 ppm metal ion of the total composition, from about 500 ppm to about 15,000 ppm or from about 3,000 ppm to about 10,000 ppm. This is the total amount of metal ions (stannous, zinc, copper and mixtures thereof) for delivery to the tooth surface.

Dentifrices containing stannous salts, such as stannous fluoride and stannous chloride, are described in U.S. Patent 5,004,597 to Majeti et al. Other descriptions of stannous salts are found in U.S. Patent 5,578,293 issued to Prencipe et al. and in U.S. Patent 5,281,410 issued to Lukacovic et al. In addition to the stannous ion source, ingredients needed to stabilize the stannous may be included, such as those described in Majeti et al. and Prencipe et al.

Stannous salts useful herein include stannous fluoride and stannous chloride dihydrate, stannous acetate, stannous tartrate and sodium stannous citrate. Examples of suitable zinc ion sources are zinc oxide, zinc sulfate, zinc chloride, zinc citrate, zinc lactate, zinc gluconate, zinc malate, zinc tartrate, zinc carbonate, zinc phosphate, and other salts listed in U.S. Pat. No 4,022,880. Examples of suitable copper ion sources are listed in U.S. Pat. No. 5,534,243 and include the chloride, sulfate, gluconate and glycinate salts. The combined metal ion source(s) will typically be present in an amount of from about 0.05% to about 11%, by weight of the final composition, from about 0.5 to about 7%, or from about 1% to about 5%. The stannous salts will typically be present in an amount of from about 0.1 to about 7%, from about 1% to about 5%, or from about 1.5% to about 3% by weight of the total composition. The amount of zinc or copper salts used in the present invention typically ranges from about 0.01 to about 5%, from about 0.05 to about 4%, or from about 0.1 to about 3.0%. Preferred metal ion sources include stannous fluoride, stannous chloride, stannous chloride dihydrate, zinc citrate, zinc lactate, zinc sulfate, zinc chloride, zinc acetate, zinc oxide, copper sulfate, and copper gluconate.

The present compositions may additionally comprise other orally-effective antimicrobial agents including non-cationic agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides, essential oils; enzymes such as endoglycosidase, papain, dextranase, mutanase, and mixtures thereof. The level of other antimicrobial agent will also depend on the type of antimicrobial agent and other factors and typically will be from about 0.01% to about 5.0%, by weight of the composition.

Antimicrobially-effective essential oils include one or more of flavor/fragrance chemicals such as citral, neral, geranial, geraniol, nerol, eucalyptol, eugenol, eugenyl acetate, carvacrol, thymol, o-cymen-5-ol (isopropylmethylphenol, IPMP), farnesol, benzyl alcohol, benzaldehyde, hinokitiol (isopropyltropolone), terpinene-4-ol, zingerone, allyl isothiocyanate, dipentene, α-pinene, β-pinene, menthol, methyl salicylate, anethole, carvone, limonene, ocimene, n-decyl alcohol, citronellal, citronellol, methyl acetate, citronellyl acetate, methyl eugenol, linalool, ethyl linalool, camphor, safrole, chlorothymol, guaiacol, phenol, phenyl salicylate , cinnamic acid, guaiacol, isoeugenol, dihydroeugenol, vanillyl butyl ether, 5-propenylguaethol, 4-ethyl-2-methoxyphenol, 4-allyl-2-methoxyphenol acetate, and 4-methyl guaiacol. Natural sources of these chemicals may be used. The selection of the essential oils to is based on demonstration of their activity against microorganisms known to be involved in undesirable oral cavity conditions such as gingivitis, periodontal disease and oral malodor. For example, useful herein is a blend of essential oils comprising at least two components, a first component selected from acyclic or non-ring structures such as citral, neral, geranial, geraniol, nerol or derivatives thereof and a second component selected from ring-containing structures such as eucalyptol, eugenol, carvacrol or derivatives thereof. These essential oil blends are described in commonly-assigned patent application published as US20080253976A1. The essential oil blend is used at a level of at least about 0.02% by weight of the composition to provide effective antimicrobial activity.

The above essential oil chemicals are preferred for use herein for their antimicrobial activity and use as flavorants. In addition, a number of the above flavor aldehydes and ketones are useful as anti-stain agents as described in our co-filed patent application entitled REDUCTION OF TOOTH STAINING DERIVED FROM CATIONIC ANTIMICROBIALS.

Volatile Oil Loading

The total level of volatile oils, i.e., flavor oils, other essential oils and performance oils such as sensate s in the composition will be at least about 0.05% up to about 5%. Typically for mouth rinses, the range will be from about 0.1% to about 1 % in order to create a more flavorful experience via higher impact/intensity and inclusion of a variety of flavor types. Formulating clear rinses wherein the flavor oils are solubilized (i.e., micellar solutions) are limited by the level and type of oil that can be used. For example, spearmints and peppermints are less soluble than wintergreens and cinnamons, and the latter are easier to formulate into a typical clear rinse. However, it is a challenge to formulate an optimum experience with peppermint or spearmint in a clear rinse based on solubility. Peppermint and spearmint are the most common and globally acceptable flavors for oral care compositions. Emulsions allow ultimate flexibility in being able to use the more insoluble flavor oils and other insoluble sensate materials such as coolants.

Solvents

The present emulsions will desirably include none or low levels of solvents such as ethanol and other co-solvents. From 0 to about 10% is the preferred ethanol level based on cost, taste, "alcohol burn", and the desire to have an "alcohol free" option. Examples of co-solvents that may be used include glycerin, propylene glycol, and polyethylene glycol at levels of up to about 20%.

Electrolytes

Electrolyte, particularly those supplying multivalent ions, have the potential to negatively affect the bioavailabilty of the quaternary ammonium agent and "crash" or cause the emulsion phases to separate, particularly when the quaternary ammonium agent such as CPC is the main emulsifier. However, many electrolytes can be valuable in providing aesthetic or functional benefits. Examples include fluoride salts used as anticaries agent, pyrophosphate and other phosphate salts used as anticalculus and anti-stain agent and saccharin used as sweetener. Emulsions allow some flexibility in allowing the addition of electrolytes but require careful balancing of the amount and species of the electrolyte to provide the benefit without crashing the emulsion or negatively affecting CPC bioavailability.

Co-Surfactants and Emulsifying Agents

Co-surfactants can aid in stabilization of emulsions but have the potential to negatively affect the bioavailability of the antimicrobial quaternary ammonium agent such as CPC, which also functions as surfactant and emulsifying agent. Low HLB (<7) surfactants typically will generally not affect CPC bioavailability to a great extent because the surfactant will primarily be in the oil phase and will have limited interaction with CPC. Higher HLB surfactants can affect bioavailabilty to a greater extent. Examples of suitable co-surfactants are nonionic ethoxylated linear alcohol surfactants having 18 or more carbons in the alcohol chain, about 35 or more EO (ethylene oxide) units and molecular weight between about 2,000 to about 15,000. These surfactants are described in co-filed application entitled REDUCTION OF TOOTH STAINING DERIVED FROM CATIONIC ANTIMICROBIALS, as providing an anti-stain benefit without compromising the bioavailability of CPC.

Cationic surfactants useful in the present invention include derivatives of aliphatic quaternary ammonium compounds described above as antimicrobial agents such as lauryl trimethylammonium chloride; cetyl pyridinium chloride; cetyl trimethylammonium bromide; di-isobutylphenoxyethyl-dimethylbenzylammonium chloride; cocoalkyl trimethylammonium chloride; cetyl pyridinium fluoride; etc. The quaternary ammonium fluorides having detergent properties are described in U.S. Patent 3,535,421 to Briner et al.

The present compositions will be essentially free of anionic, nonionic or amphoteric surfactants, which have been found to have a negative effect on bioavailability of the quaternary ammonium antimicrobial and thus, its bactericidal efficacy. By "essentially free of anionic, nonionic or amphoteric surfactants" as used herein, means the composition may comprise only such an amount of surfactant, which will not substantially impair the activity of the quaternary ammonium antimicrobial agent. Generally this means the composition will contain less than about 0.1% total additional surfactant by weight of the composition. Preferably the composition will contain less than 0.05%, more preferably less than 0.01% and most preferably 0% of anionic surfactant or amphoteric surfactant. Preferably the composition will contain less than about 0.1%, more preferably less than 0.06% of nonionic surfactant.

If present in the composition, preferred nonionic surfactants include poloxamers (sold under the trade name Pluronic). Other suitable nonionic surfactants include polyoxyethylene fatty alcohol ethoxylates, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, sorbitan esters (sold under trade name Tweens), and mixtures of such materials.

If present, amphoteric surfactants that may be used include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate. Examples are betaine surfactants such as disclosed in U.S. Patent 5,180,577 to Polefka et al. Typical alkyl dimethyl betaines include decyl betaine or 2-(N-decyl-N,N-dimethylammonio) acetate, coco betaine, myristyl betaine, palmityl betaine, lauryl betaine, cetyl betaine, cetyl betaine, stearyl betaine, etc. The amidobetaines are exemplified by cocoamidoethyl betaine, cocoamidopropyl betaine, lauramidopropyl betaine and the like.

If present, suitable anionic surfactants include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate and sodium coconut monoglyceride sulfonates are examples of anionic surfactants of this type. Other suitable anionic surfactants are sarcosinates, such as sodium lauroyl sarcosinate, taurates, sodium lauryl sulfoacetate, sodium lauroyl isethionate, sodium laureth carboxylate, and sodium dodecyl benzenesulfonate.

Emulsifying agents can also aid in stabilizing the present emulsions. Examples of emulsifying agents include poloxamers described above as a nonionic surfactant, which may also function as binder, stabilizer, and other related functions. Poloxamers are difunctional block-polymers terminating in primary hydroxyl groups with molecular weights ranging from 1,000 to above 15,000. Poloxamers are sold under the tradename of Pluronics and Pluraflo by BASF, such as Poloxamer 407 and Pluraflo L4370. Other suitable emulsifying agents include the polyacrylic acid Pemulen® series available from B.F. Goodrich; Vitamin E acetate; Vitamin E succinate and pegylated Vitamin E.

Other optional components collectively referred to as orally acceptable carrier materials are described in the following paragraphs.

Orally Acceptable Carrier Materials

The orally acceptable carrier materials comprise one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy. In particular, the carrier materials should not have a negative effect on the stability of the present emulsions, the bioavailability of the cationic antimicrobials or on the antistaining activity of the anti-stain agents used herein.

The carriers or excipients used in the present invention can include the usual and conventional components of mouthwashes or mouth rinses, mouth sprays, dentifrices, non-abrasive gels, subgingival gels, chewing gums, lozenges and breath mints as more fully described hereinafter.

The choice of a carrier to be used is basically determined by the way the composition is to be introduced into the oral cavity. Preferred embodiments of the subject invention are liquid products, including mouthwashes or mouth rinses, mouth sprays, dental solutions and irrigation fluids. Mouthwash, rinse or mouth spray carrier materials are disclosed in, e.g., U.S. Pat. No. 3,988,433 to Benedict. Components of such mouthwashes and mouth sprays typically include one or more of water (from about 45% to about 95%), ethanol (from about 0% to about 25%), a humectant (from about 0% to about 50%), a surfactant (from about 0.01% to about 7%), a flavoring agent (from about 0.04% to about 2%), a sweetening agent (from about 0.1% to about 3%), and a coloring agent (from about 0.001% to about 0.5%). Such mouthwashes and mouth sprays may also include one or more of an anticaries agent (from about 0.05% to about 0.3% as fluoride ion) and an anticalculus agent (from about 0.1% to about 3%). Components of dental solutions generally include one or more of water (from about 90% to about 99%), preservative (from about 0.01% to about 0.5%), thickening agent (from 0% to about 5%), flavoring agent (from about 0.04% to about 2%), sweetening agent (from about 0.1% to about 3%), and surfactant (from 0% to about 5%).

The present oil-in-water emulsions may also be incorporated into other oral care forms such as dentifrices or toothpastes, for example as the antimicrobial and/or flavor component.

Carrier materials for toothpaste, tooth gel or the like include abrasive materials, sudsing agents, binders, humectants, flavoring and sweetening agents, etc. as disclosed in e.g., U.S. Pat. No. 3,988,433 to Benedict. Carrier materials for biphasic dentifrice formulations are disclosed in U.S. Pat. Nos. 5,213,790, issued May 23, 1993, 5,145,666, and 5,281,410 all to Lukacovic et al. and in U. S. Pat. Nos. 4,849,213 and 4,528,180 to Schaeffer. Lozenge carrier materials typically include a candy base; chewing gum carrier materials include a gum base, flavoring and sweetening agents, as in, e.g., U.S. Pat. No. 4,083,955 to Grabenstetter et al. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910 both to Damani. Carriers suitable for the preparation of compositions of the present invention are well known in the art. Their selection will depend on secondary considerations like taste, cost, and shelf stability, etc.

The compositions of the present invention may also be in the form of non-abrasive gels and subgingival gels, which may be aqueous or essentially non-aqueous. In still another aspect, the invention provides a dental implement impregnated with the present composition. The dental implement comprises an implement for contact with teeth and other tissues in the oral cavity, said implement being impregnated with the present composition. The dental implement can be impregnated fibers including dental floss or tape, chips, strips, films and polymer fibers.

In one embodiment, the compositions of the subject invention are in the form of dentifrices, such as toothpastes, tooth gels and tooth powders. Components of such toothpaste and tooth gels generally include one or more of a dental abrasive (from about 6% to about 50%), a surfactant (from about 0.5% to about 10%), a thickening agent (from about 0.1% to about 5%), a humectant (from about 10% to about 55%), a flavoring agent (from about 0.04% to about 2%), a sweetening agent (from about 0.1 % to about 3 %), a coloring agent (from about 0.01% to about 0.5%) and water (from about 2% to about 45%). Such toothpaste or tooth gel may also include one or more of an anticaries agent (from about 0.05% to about 0.3% as fluoride ion) and an anticalculus agent (from about 0.1% to about 13%). Tooth powders, of course, contain substantially all non-liquid components.

Types of orally acceptable carrier materials or excipients, which may optionally be included in compositions of the present invention, along with specific non-limiting examples, are described in the following paragraphs.

Desensitizing Agent

The present compositions may optionally contain a dentinal desensitizing agent such as salts of potassium, calcium, strontium and tin including nitrate, chloride, fluoride, phosphates, pyrophosphate, polyphosphate, citrate, oxalate and sulfate.

Anti-inflammatory Agents

Anti-inflammatory agents may also be used in the present emulsion compositions to further enhance efficacy against bacteria-mediated conditions, specifically dental plaque, gingivitis and periodontal disease. In addition to bacterial infection, periodontal disease may involve one or more of the following conditions: inflammation of the gingiva, formation of periodontal pockets, bleeding and/or pus discharge from the periodontal pockets, resorption of alveolar bone, loose teeth and loss of teeth. Bacteria present in dental plaque which forms on the surface of the teeth and in the periodontal pocket contribute to both the initiation and progress of periodontal disease. Severe periodontal disease involves the destruction of periodontal tissue, which is primarily caused by the indirect effects mediated by the host's reaction to the bacteria in the periodontium and gingival sulcus, specifically inflammation which is a nonspecific cellular and biochemical process involving multiple pro-inflammatory agents. Once inflammation starts, the process can self-propagate even when the causative agent, i.e., bacteria, are removed. Therefore, an anti-bacterial (or anti-microbial) agents, such as stannous, zinc, CPC and peroxide, in combination with an anti-inflammatory agent would be a more effective therapy for gingivitis and periodontal disease than the conventional method of using anti-bacterial agents alone.

Anti-inflammatory agents useful herein include those described in WO2008/057136A1 to Procter & Gamble, Doyle, et al. The assays described therein identified agents having potent anti-inflammatory activity including vitamin compounds such as riboflavin, riboflavin phosphate, folic acid, cyanocobalamin (vitamin B12), and menadione (vitamin K3); curcuminoids such as curcumin, demethoxycurcumin, bismethoxycurcumin and tetrahydrocurcumin; oils and extracts from spices and botanicals such as clove, cinnamon, cassia, ginger, basil, coriander, cilantro and allspice which contain active compounds including cinnamaldehyde, cinnamic acid, guaiacol and derivatives such as eugenol, isoeugenol, dihydroeugenol, vanillyl butyl ether, vanillin (4-formyl-guaiacol), 5-propenylguaethol, 4-ethyl-2-methoxyphenol, 4-allyl-2-methoxyphenol acetate, and 4-methyl guaiacol; oils or extracts of thyme, oregano and sage containing thymol, carvacrol and carvacryl ethyl ether; neem oil; flavonoids and flavones such as baicalein, baicalin, wogonoside, wogonin, and quercetin; phenolics from plant sources such as tea and cranberry including catechin, gallocatechin gallate, epicatechin (EC), epigallocatechin (EGC), epigallocatechin gallate (EGCG), epicatechin gallate (ECG), theaflavine, thearubigins, anthocyanidins/proanthocyanidins and anthocyanins (e.g., cyanidin, delphinidin, pelargonidin, peonidin, malvidin and petunidin); tannic acid; gallic acid; ellagic acid; ellagitannins; hexamidine; and berberine.

Additional useful agents that have been identified as having anti-inflammatory activity, include menthyl anthranilate, (used commercially in lip balm as a sunscreen agent); hexyl isobutyrate (grape flavorant); 4-hydroxybenzaldehyde (flavor component of vanilla extract); a broad group of polyphenols including resveratrol (component of red wines, found in grape skins), isoliquertigenin (found in licorice), apigenin (found in chamomile), pratol (found in red clover), 4'-methoxyflavone, 8-methyl-4'-methoxyflavone and 6-methyl-4'-methoxyflavone. Additional agents with inhibitory activity include: brazilin and quercetin; green tea and Echinacea extracts; cinnamon; curcumin; caffeic acid phenethyl ester; preparations of bee propolis; silymarin; fisetin; quercetin; luteoline; apigenin; diosmetin; and a wide variety of plant-derived chemicals: flavonoids, isoflavonoids and other phenolics (e.g., myricetin, kaempferol, luteolin, hesperitin, naringenin, pterostilbene, rutin, rosmarinic acid, glabridin); carotenoids (e.g., lycopene, lutein, zeaxanthin, astazaxanthin, beta-carotene); limonoids and terpenoids (e.g., limonene, geraniol, farnesol); phytosterols (e.g., beta-sitosterol, stigmasterol, campesterol, ursolic acid); allicin; chlorogenic acid; ferulic acid; emodin; isothiocyanates (e.g., sulphoraphane); N-acetyl cysteine; phytic acid; and betaine.

Still other anti-inflammatory agents that may be used include lipoxygenase inhibitors, such as nordihydroguaiaretic acid; cyclo-oxygenase inhibitors such as flurbiprofen; and non-steroidal anti-inflammatory agents such as aspirin, ketorolac, flurbiprofen, ibuprofen, naproxen, indomethacin, aspirin, ketoprofen, piroxicam and meclofenamic acid, rofecoxib, and celecoxib.

Of the above anti-inflammatory agents, preferred are the natural essential oil materials already known to be safe for ingestion. For example, a number of the polyphenols above that have potent anti-inflammatory activity are natural components of tea (*Camellia sinensis*) which are regularly consumed by humans. In addition many of these essential oil materials also have antimicrobial potency. The anti-inflammatory agent will typically be present at from about 0.001% to about 10% by weight of the composition.

Furthermore, antimicrobial agents such as CPC, stannous fluoride, zinc citrate, zinc lactate, zinc oxide, copper sulfate, copper gluconate and triclosan have been found to also provide anti-inflammatory activity. Thus, the anti-gingivitis benefit from these antimicrobial actives is mediated in part by their anti-inflammatory action and prevention of tissue destruction in addition to their anti-bacterial action.

Anticalculus Agent

The present compositions may optionally include an anticalculus agent, such as a pyrophosphate salt as a source of pyrophosphate ion. The pyrophosphate salts useful in the present compositions include the dialkali metal pyrophosphate salts, tetraalkali metal pyrophosphate salts, and mixtures thereof. Disodium dihydrogen pyrophosphate ($Na_2H_2P_2O_7$), tetrasodium pyrophosphate ($Na_4P_2O_7$), and tetrapotassium pyrophosphate ($K_4P_2O_7$) in their unhydrated as well as hydrated forms are the preferred species. In compositions of the present invention, the pyrophosphate salt may be present in one of three ways: predominately dissolved, predominately undissolved, or a mixture of dissolved and undissolved pyrophosphate.

Compositions comprising predominately dissolved pyrophosphate refer to compositions where at least one pyrophosphate ion source is in an amount sufficient to provide at least about 1.0% free pyrophosphate ions. The amount of free pyrophosphate ions may be from about 1% to about 15%, from about 1.5% to about 10% in one embodiment, and from about 2% to about 6% in another embodiment. Free pyrophosphate ions may be present in a variety of protonated states depending on the pH of the composition.

Compositions comprising predominately undissolved pyrophosphate refer to compositions containing no more than about 20% of the total pyrophosphate salt dissolved in the composition, or less than about 10% of the total pyrophosphate dissolved in the composition. Tetrasodium pyrophosphate salt is a preferred pyrophosphate salt in these compositions. Tetrasodium pyrophosphate may be the anhydrous salt form or the decahydrate form, or any other species stable in solid form in the dentifrice compositions. The salt is in its solid particle form, which may be its crystalline and/or amorphous state, with the particle size of the salt preferably being small enough to be aesthetically acceptable and readily soluble during use. The amount of pyrophosphate salt useful in making these compositions is any tartar control effective amount, generally from about 1.5% to about 15%, from about 2% to about 10%, or from about 3% to about 8%, by weight of the dentifrice composition.

Compositions may also comprise a mixture of dissolved and undissolved pyrophosphate salts. Any of the above mentioned pyrophosphate salts may be used.

The pyrophosphate salts are described in more detail in Kirk-Othmer Encyclopedia of Chemical Technology, Third Edition, Volume 17, Wiley-Interscience Publishers (1982).

Optional agents to be used in place of or in combination with the pyrophosphate salt include such known materials as synthetic anionic polymers, including polyacrylates and copolymers of maleic anhydride or acid and methyl vinyl ether (e.g., Gantrez), as described, for example, in U.S. Pat. No. 4,627,977, to Gaffar et al., as well as, e.g., polyamino propane sulfonic acid (AMPS), diphosphonates (e.g., EHDP; AHP), polypeptides (such as polyaspartic and polyglutamic acids), and mixtures thereof.

Fluoride Ion Source

It is common to have a water-soluble fluoride compound present in dentifrices and other oral compositions in an amount sufficient to give a fluoride ion concentration in the composition, and/or when it is used of from about 0.0025% to about 5.0% by weight or from about 0.005% to about 2.0% by weight, to provide anticaries effectiveness. A wide variety of fluoride ion-yielding materials can be employed as sources of soluble fluoride in the present compositions. Examples of suitable fluoride ion-yielding materials are found in U.S. Patent No. 3,535,421, October 20, 1970 to Briner et al. and U.S. Patent No. 3,678,154, July 18, 1972 to Widder et al. Representative fluoride ion sources include: stannous fluoride, sodium fluoride, potassium fluoride, sodium monofluorophosphate, indium fluoride, amine fluoride and many others. Stannous fluoride and sodium fluoride are among preferred sources, as well as mixtures thereof.

Abrasives

Dental abrasives useful in the compositions of the subject invention include many different materials. The material selected must be one which is compatible within the composition of interest and does not excessively abrade dentin. Suitable abrasives include, for example, silicas including gels and precipitates, insoluble sodium polymetaphosphate, hydrated alumina, calcium carbonate, dicalcium orthophosphate dihydrate, calcium pyrophosphate, tricalcium phosphate, calcium polymetaphosphate, and resinous abrasive materials such as particulate condensation products of urea and formaldehyde.

Another class of abrasives for use in the present compositions is the particulate thermosetting polymerized resins as described in U.S. Pat. No. 3,070,510 issued to Cooley and Grabenstetter. Suitable resins include, for example, melamines, phenolics, ureas, melamine-ureas, melamine-formaldehydes, urea-formaldehyde, melamine-urea-formaldehydes, cross-linked epoxides, and cross-linked polyesters.

Silica dental abrasives of various types are preferred because of their unique benefits of exceptional dental cleaning and polishing performance without unduly abrading tooth enamel or dentine. The silica abrasive polishing materials herein, as well as other abrasives, generally have an average particle size ranging between about 0.1 to about 30 microns, and preferably from about 5 to about 15 microns. The abrasive can be precipitated silica or silica gels such as the silica xerogels described in Pader et al., U.S. Patent 3,538,230 and DiGiulio, U.S. Patent 3,862,307. Examples include the silica xerogels marketed under the trade name "Syloid" by the W.R. Grace & Company, Davison Chemical Division and precipitated silica materials such as those marketed by the J. M. Huber Corporation under the trade name, Zeodent®, particularly the silicas carrying the designation Zeodent® 119, Zeodent® 118, Zeodent® 109 and Zeodent® 129. The types of silica dental abrasives useful in the toothpastes of the present invention are described in more detail in Wason, U.S. Patent 4,340,583; and in commonly-assigned US Pat. Nos. 5,603,920; 5,589,160; 5,658,553; 5,651,958; and 6,740,311.

Mixtures of abrasives can be used such as mixtures of the various grades of Zeodent® silica abrasives listed above. The total amount of abrasive in dentifrice compositions of the subject invention typically range from about 6% to about 70% by weight; toothpastes generally contain from about 10% to about 50% of abrasives, by weight of the composition. Dental solution, mouth spray, mouthwash and non-abrasive gel compositions of the subject invention typically contain little or no abrasive.

Tooth Substantive Agent

The present invention may include a tooth substantive agent such as polymeric surface active agents (PMSA's), which are polyelectrolytes, more specifically anionic polymers. The PMSA's contain anionic groups, e.g., phosphate, phosphonate, carboxy, or mixtures thereof, and thus, have the capability to interact with cationic or positively charged entities. The "mineral" descriptor is intended to convey that the surface activity or substantivity of the polymer is toward mineral surfaces such as calcium phosphate minerals or teeth.

PMSA's are useful in the present compositions because of their stain prevention benefit. The PMSA's may provide a stain prevention benefit because of their reactivity or substantivity to mineral surfaces, resulting in desorption of portions of undesirable adsorbed pellicle proteins, in particular those associated with binding color bodies that stain teeth, calculus development and attraction of undesirable microbial species. The retention of these PMSA's on teeth can also prevent stains from accruing due to disruption of binding sites of color bodies on tooth surfaces.

The ability of PMSA's to bind stain promoting ingredients of oral care products, for example, stannous ions and cationic antimicrobials, is also believed to be helpful. The PMSA will also provide tooth surface conditioning effects which produce desirable effects on surface thermodynamic properties and surface film properties, which impart improved clean feel aesthetics both during and most importantly, following rinsing or brushing. Many of these polymeric agents are also known or expected to provide tartar control benefits when applied in oral compositions, hence providing improvement in both the appearance of teeth and their tactile impression to consumers.

The polymeric mineral surface active agents include an agent which will have a strong affinity for the tooth surface, deposit a polymer layer or coating on the tooth surface and produce the desired surface modification effects. Suitable examples of such polymers are polyelectrolytes such as condensed phosphorylated polymers; polyphosphonates; copolymers of phosphate- or phosphonate-containing monomers or polymers with other monomers such as ethylenically unsaturated monomers and amino acids or with other polymers such as proteins, polypeptides, polysaccharides, poly(acrylate), poly(acrylamide), poly(methacrylate), poly(ethacrylate), poly(hydroxyalkylmethacrylate), poly(vinyl alcohol), poly(maleic anhydride), poly(maleate) poly(amide), poly(ethylene amine), poly(ethylene glycol), poly(propylene glycol), poly(vinyl acetate) and poly(vinyl benzyl chloride); polycarboxylates and carboxy-substituted polymers; and mixtures thereof. Suitable polymeric mineral surface active agents include the carboxy-substituted alcohol polymers described in U.S. Patent Nos. 5,292,501; 5,213,789, 5,093,170, 5,009,882; and 4,939,284; all to Degenhardt et al. and the diphosphonate-derivatized polymers in U.S. patent 5,011,913 to Benedict et al; the synthetic anionic polymers including polyacrylates and copolymers of maleic anhydride or acid and methyl vinyl ether (e.g., Gantrez), as described, for example, in U.S. Patent 4,627,977, to Gaffar et al. Diphosphonate modified polyacrylic acid is another example. Polymers with activity must have sufficient surface binding propensity to desorb pellicle proteins and remain affixed to enamel surfaces. For tooth surfaces, polymers with end or side chain phosphate or phosphonate functions are preferred although other polymers with mineral binding activity may prove effective depending upon adsorption affinity.

Additional examples of suitable phosphonate containing polymeric mineral surface active agents include the geminal diphosphonate polymers disclosed as anticalculus agents in US 4,877,603 to Degenhardt et al; phosphonate group containing copolymers disclosed in US 4,749,758 to Dursch et al. and in GB 1,290,724 (both assigned to Hoechst) suitable for use in detergent and cleaning compositions; and the copolymers and cotelomers disclosed as useful for applications including scale and corrosion inhibition, coatings, cements and ion-exchange resins in US 5,980,776 to Zakikhani et al. and US 6,071,434 to Davis et al. Additional polymers include the water-soluble copolymers of vinylphosphonic acid and acrylic acid and salts thereof disclosed in GB 1,290,724 wherein the copolymers contain from about 10% to about 90% by weight vinylphosphonic acid and from about 90% to about 10% by weight acrylic acid, more particularly wherein the copolymers have a weight ratio of vinylphosphonic acid to acrylic acid of 70% vinylphosphonic acid to 30% acrylic acid; 50% vinylphosphonic acid to 50% acrylic acid; or 30% vinylphosphonic acid to 70% acrylic acid. Other suitable polymers include the water soluble polymers disclosed by Zakikhani and Davis prepared by copolymerizing diphosphonate or polyphosphonate monomers having one or more unsaturated C=C bonds (e.g., vinylidene-1,1-diphosphonic acid and 2-(hydroxyphosphinyl)ethylidene-1,1-diphosphonic acid), with at least one further compound having unsaturated C=C bonds (e.g., acrylate and methacrylate monomers). Suitable polymers include the diphosphonate/acrylate polymers supplied by Rhodia under the designation ITC 1087 (Average MW 3000-60,000) and Polymer 1154 (Average MW 6000-55,000).

Suitable PMSA's will be stable and compatible with other components of the oral care composition such as ionic fluoride, cationic antimicrobials and metal ions, and are stable to hydrolysis in high water content formulations, thus permitting a simple single phase dentifrice or mouth rinse formulation. If the PMSA does not have these stability and compatibility properties, one option is a dual phase formulation with the PMSA separated from the fluoride or other incompatible component. Another option is to formulate non-aqueous, essentially non-aqueous or limited water compositions to minimize reaction between the PMSA and other components.

A preferred PMSA is a polyphosphate. A polyphosphate is generally understood to consist of two or more phosphate molecules arranged primarily in a linear configuration, although some cyclic derivatives may be present. Preferred polyphosphates are those having around three or more phosphate groups so that surface adsorption at effective concentrations produces sufficient non-bound phosphate functions, which enhance the anionic surface charge as well as hydrophilic character of the surfaces. The polyphosphate salts desired include tripolyphosphate, tetrapolyphosphate and hexametaphosphate, among others. Polyphosphates larger than tetrapolyphosphate usually occur as amorphous glassy materials. Preferred in this invention are the linear polyphosphates having the formula: $XO(XPO_3)_nX$, wherein X is sodium, potassium or ammonium and n averages from about 3 to about 125. Preferred polyphosphates are those having n averaging from about 6 to about 21, such as those commercially known as Sodaphos (n≈6), Hexaphos (n≈13), and Glass H (n≈21) and manufactured by FMC Corporation and Astaris. These polyphosphates may be used alone or in combination. Some polyphosphates are susceptible to hydrolysis in high water formulations at acid pH, particularly below pH 5. Thus it is preferred to use longer-chain polyphosphates, such as Glass H having an average chain length of about 21. Such longer-chain polyphosphates when undergoing hydrolysis, produce shorter-chain polyphosphates which are still effective to deposit onto teeth and provide a stain preventive benefit.

Other polyphosphorylated compounds may be used in addition to or instead of the polyphosphate, in particular polyphosphorylated inositol compounds such as phytic acid, myo-inositol pentakis(dihydrogen phosphate); myo-inositol tetrakis(dihydrogen phosphate), myo-inositol trikis(dihydrogen phosphate), and an alkali metal, alkaline earth metal or ammonium salt thereof. Preferred herein is phytic acid, also known as myo-inositol 1,2,3,4,5,6-hexakis (dihydrogen phosphate) or inositol hexaphosphoric acid, and its alkali metal, alkaline earth metal or ammonium salts. Herein, the term "phytate" includes phytic acid and its salts as well as the other polyphosphorylated inositol compounds.

The amount of tooth substantive agent may be from about 0.1% to about 35% by weight of the total oral composition. In dentifrice formulations, the amount is typically from about 2% to about 30%, from about 5% to about 25%, or from about 6% to about 20%. In mouth rinse compositions, the amount of tooth substantive agent is typically from about 0.1% to 5% or from about 0.5% to about 3%.

In addition to creating surface modifying effects, the tooth substantive agent may also function to solubilize insoluble salts. For example, Glass H has been found to solubilize insoluble stannous salts. Thus, in compositions containing stannous fluoride for example, Glass H contributes to decreasing the stain promoting effect of stannous.

Chelating agents

Another optional agent is a chelating agent, also called sequestrants, such as gluconic acid, tartaric acid, citric acid and pharmaceutically-acceptable salts thereof. Chelating agents are able to complex calcium found in the cell walls of the bacteria. Chelating agents can also disrupt plaque by removing calcium from the calcium bridges which help hold this biomass intact. However, it is not desired to use a chelating agent which has an affinity for calcium that is too high, as this may result in tooth demineralization, which is contrary to the objects and intentions of the present invention. Suitable chelating agents will generally have a calcium binding constant of about $10^1$ to $10^5$ to provide improved cleaning with reduced plaque and calculus formation. Chelating agents also have the ability to complex with metallic ions and thus aid in preventing their adverse effects on the stability or appearance of products. Chelation of ions, such as iron or copper, helps retard oxidative deterioration of finished products.

Examples of suitable chelating agents are sodium or potassium gluconate and citrate; citric acid/alkali metal citrate combination; disodium tartrate; dipotassium tartrate; sodium potassium tartrate; sodium hydrogen tartrate; potassium hydrogen tartrate; sodium, potassium or ammonium polyphosphates and mixtures thereof. The amounts of chelating agent suitable for use in the present invention will typically be from about 0.1% to about 2.5%, from about 0.5% to about 2.5%, or from about 1.0% to about 2.5%.

Still other chelating agents suitable for use in the present invention are the anionic polymeric polycarboxylates. Such materials are well known in the art, being employed in the form of their free acids or partially or preferably fully neutralized water soluble alkali metal (e.g. potassium and preferably sodium) or ammonium salts. Examples are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 1,000,000. These copolymers are available for example as Gantrez AN 139 (M.W. 500,000), AN 119 (M.W. 250,000) and S-97 Pharmaceutical Grade (M.W. 70,000), of GAF Chemicals Corporation.

Other operative polymeric polycarboxylates include the 1:1 copolymers of maleic anhydride with ethyl acrylate, hydroxyethyl methacrylate, N-vinyl-2-pyrrolidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Additional operative polymeric polycarboxylates are disclosed in U.S. Pat. Nos. 4,138,477 and 4,183,914 to Gaffar et al. and include copolymers of maleic anhydride with styrene, isobutylene or ethyl vinyl ether; polyacrylic, polyitaconic and polymaleic acids; and sulfoacrylic oligomers of M.W. as low as 1,000 available as Uniroyal ND-2.

Thickening Agents

In preparing toothpaste or gels, thickening agents are added to provide a desirable consistency to the composition, to provide desirable active release characteristics upon use, to provide shelf stability, and to provide stability of the composition, etc. Suitable thickening agents include one or a combination of carboxyvinyl polymers, carrageenan, hydroxyethyl cellulose (HEC), natural and synthetic clays (e.g., Veegum and laponite) and water soluble salts of cellulose ethers such as sodium carboxymethylcellulose (CMC) and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture.

Suitable carboxyvinyl polymers useful as thickening or gelling agents include carbomers which are homopolymers of acrylic acid crosslinked with an alkyl ether of pentaerythritol or an alkyl ether of sucrose. Carbomers are commercially available from B.F. Goodrich as the Carbopol® series, including Carbopol 934, 940, 941, 956, and mixtures thereof.

Thickening agents are typically present in an amount from about 0.1% to about 15%, from about 2% to about 10%, or from about 4% to about 8%, by weight of the total toothpaste or gel composition, can be used. Higher concentrations may be used for chewing gums, lozenges and breath mints, sachets, non-abrasive gels and subgingival gels.

Humectants

Another optional carrier material of the present compositions is a humectant. The humectant serves to keep toothpaste compositions from hardening upon exposure to air, to give compositions a moist feel to the mouth, and, for particular humectants, to impart desirable sweetness of flavor to toothpaste compositions. The humectant, on a pure humectant basis, generally comprises from about 0% to about 70% or from about 5% to about 25%, by weight of the compositions herein. Suitable humectants for use in compositions of the subject invention include edible polyhydric alcohols such as glycerin, sorbitol, xylitol, butylene glycol, polyethylene glycol, propylene glycol and trimethyl glycine.

Flavor System

A flavor system is typically added to oral care compositions, to provide a pleasant tasting composition and to effectively mask any unpleasant taste and sensations due to certain components of the composition such as antimicrobial actives or peroxide. Pleasant tasting compositions improve user compliance to prescribed or recommended use of oral care products. The present flavor system will comprise flavor components, such as those that have been found to be relatively stable in the presence of usual oral care product actives, carrier materials or excipients. The flavor system may comprise flavor ingredients including but not limited to peppermint oil, corn mint oil, spearmint oil, oil of wintergreen, clove bud oil, cassia, sage, parsley oil, marjoram, lemon, lime, orange, *cis*-jasmone, 2,5-dimethyl-4-hydroxy-3(2H)-furanone, 5-ethyl-3-hydroxy-4-methyl-2(5H)-furanone, vanillin, ethyl vanillin, 2-methoxybenzaldehyde, benzalde-hyde; cinnamaldehyde, hexyl cinnamaldehyde, $\alpha$-methyl cinnamaldehyde, ortho-methoxy cinnamaldehyde, $\alpha$-amyl cin-namaldehydepropenyl guaethol, heliotropine, 4-*cis*-heptenal, diacetyl, methyl-$\rho$-*tert*-butyl phenyl acetate, menthol, methyl salicylate, ethyl salicylate, 1-menthyl acetate, oxanone, $\alpha$-irisone, methyl cinnamate, ethyl cinnamate, butyl cinnamate, ethyl butyrate, ethyl acetate, methyl anthranilate, *iso*-amyl acetate, iso-amyl butyrate, allyl caproate, eugenol, eucalyptol, thymol, cinnamic alcohol, octanol, octanal, decanol, decanal, phenylethyl alcohol, benzyl alcohol, $\alpha$-terpineol, linalool, limonene, citral, maltol, ethyl maltol, anethole, dihydroanethole, carvone, menthone, $\beta$-damascenone, ionone, gamma-decalactone, gamma-nonalactone, gamma-undecalactone and mixtures thereof. Generally suitable flavoring ingredients are those containing structural features and functional groups that are less prone to redox reactions. These include derivatives of flavor chemicals that are saturated or contain stable aromatic rings or ester groups. Also suitable are flavor chemicals that may undergo some oxidation or degradation without resulting in a significant change in the flavor character or profile. The flavor ingredients may be supplied in the composition as single or purified chemicals or by addition of natural oils or extracts that have preferably undergone a refining treatment to remove components that are relatively unstable and may degrade and alter the desired flavor profile, resulting in a less acceptable product from an organoleptic standpoint. Flavoring agents are generally used in the compositions at levels of from about 0.001% to about 5%, by weight of the composition.

The flavor system will typically include a sweetening agent. Suitable sweeteners include those well known in the art, including both natural and artificial sweeteners. Some suitable water-soluble sweeteners include monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin. Suitable water-soluble artificial sweeteners include soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like. Other suitable sweeteners include dipeptide based sweeteners, such as L-aspartic acid derived sweeteners, such as L-aspartyl-L-phenylalanine methyl ester (aspartame) and materials described in U.S. Pat. No. 3,492,131, L-alpha-aspartyl-N-(2,2,4,4-tetramethyl-3-thietanyl)-D-alaninamide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclobexylen)-alanine, and the like. Water-soluble sweeteners derived from naturally occurring water-soluble sweeteners, such as a chlorinated derivative of ordinary sugar (sucrose), known, for example, under the product description of sucralose as well as protein based sweeteners such as thaumatoccous danielli (Thaumatin I and II) can be used. A composition preferably contains from about 0.1% to about 10% of sweetener, by weight.

Suitable cooling agents or coolants include a wide variety of materials such as menthol and derivatives thereof. Among synthetic coolants, many are derivatives of or are structurally related to menthol, i.e., containing the cyclohexane moiety, and derivatized with functional groups including carboxamide, ketal, ester, ether and alcohol. Examples include the $\rho$-menthanecarboxamide compounds such as N-ethyl-$p$-menthan-3-carboxamide, known commercially as "WS-3", and others in the series such as WS-5, WS-11, WS-14 and WS-30. An example of a synthetic carboxamide coolant that is structurally unrelated to menthol is N,2,3-trimethyl-2-isopropylbutanamide, known as "WS-23". Additional suitable coolants include 3-1-menthoxypropane-1,2-diol known as TK-10, isopulegol (under the tradename Coolact P) and $\rho$-menthane-3,8-diol (under the tradename Coolact 38D) all available from Takasago; menthone glycerol acetal known as MGA; menthyl esthers such as menthyl acetate, menthyl acetoacetate, menthyl lactate known as Frescolat® supplied by Haarmann and Reimer, and monomenthyl succinate under the tradename Physcool from V. Mane. The terms menthol and menthyl as used herein include dextro- and levorotatory isomers of these compounds and racemic mixtures thereof. TK-10 is described in U.S. Pat. No. 4,459,425, Amano et al. WS-3 and other carboxamide cooling agents are described for example in U.S. Pat. Nos. 4,136,163; 4,150,052; 4,153,679; 4,157,384; 4,178,459 and 4,230,688. Additional N-substituted $\rho$-menthane carboxamides are described in WO 2005/049553A1 including N-(4-cyanomethylphenyl)-$\rho$-men-thanecarboxamide, N-(4-sulfamoylphenyl)-$\rho$-menthanecarboxamide, N-(4-cyanophenyl)-$\rho$-menthanecarboxamide, N-(4-acetylphenyl)-$\rho$-menthanecarboxamide, N-(4-hydroxymethylphenyl)-p-menthanecarboxamide and N-(3-hydroxy-4-methoxyphenyl)-$\rho$-menthanecarboxamide.

The flavor system may also include other sensates such as salivating agents, hydration and moisturization agents, warming agents, and numbing agents. These agents are present in the compositions at a level of from about 0.001% to about 10% or from about 0.1% to about 1%, by weight of the composition. Suitable salivating agents include Jambu® manufactured by Takasago and Optaflow® from Symrise. Examples of hydration agents include polyols such as erythritol. Suitable numbing agents include benzocaine, lidocaine, clove bud oil, and ethanol. Examples of warming agents include ethanol, capsicum and nicotinate esters, such as benzyl nicotinate.

Miscellaneous Carrier Materials

Water employed in the preparation of commercially suitable oral compositions desirably would be of low ion content and free of organic impurities. Water may comprise up to about 99% by weight of the aqueous compositions herein. These amounts of water include the free water which is added plus that which is introduced with other materials, such as with sorbitol.

The present invention may also include an alkali metal bicarbonate salt, which may serve a number of functions including effervescent, abrasive, deodorant, buffering and adjusting pH. The present composition may contain from about 0.5% to about 30%, from about 0.5% to about 15% or from about 0.5% to about 5% of an alkali metal bicarbonate such as sodium bicarbonate.

The pH of the present compositions may be adjusted through the use of buffering agents. Buffering agents, as used herein, refer to agents that can be used to adjust the pH of aqueous compositions such as mouth rinses and dental solutions typically to a range of about 3 to about 8, preferably from about 3 to about 6. Buffering agents include sodium bicarbonate, monosodium phosphate, trisodium phosphate, sodium hydroxide, sodium carbonate, sodium acid pyrophosphate, citric acid, and sodium citrate. Buffering agents are typically included at a level of from about 0.5% to about 10%, by weight of the present compositions.

Titanium dioxide may also be added to the present composition to add opacity to the compositions. Titanium dioxide generally comprises from about 0.25% to about 5% by weight of dentifrice compositions.

Other optional agents that may be used in the present compositions include dimethicone copolyols selected from alkyl- and alkoxy-dimethicone copolyols, such as C12 to C20 alkyl dimethicone copolyols and mixtures thereof. An example is cetyl dimethicone copolyol marketed under the trade name Abil EM90. The dimethicone copolyols aid in providing positive tooth feel benefits and may be present at a level of from about 0.01% to about 25%.

Method of Use

The present invention also relates to the use of the compositions for control of staining and for controlling bacterial activity in the oral cavitywhich cause undesirable conditions including plaque, caries, calculus, gingivitis, and periodontal disease. The benefits of these compositions may increase over time when the composition is used repeatedly.

The method of use or treatment herein comprises contacting a subject's dental enamel surfaces and mucosa in the mouth with the oral compositions according to the present invention. The method may comprise brushing with a dentifrice or rinsing with a dentifrice slurry or mouth rinse. Other methods include contacting the topical oral gel, denture product, mouthspray, or other form with the subject's teeth and oral mucosa. The subject may be any person or animal in need of oral care. By animal is meant to include household pets or other domestic animals, or animals kept in captivity.

For example, a method of treatment may include a person brushing a dog's teeth with one of the dentifrice compositions. Another example would include rinsing a cat's mouth with an oral composition for a sufficient amount of time to see a benefit. Pet care products such as chews and toys may be formulated to contain the present oral compositions. The composition may be incorporated into a relatively supple but strong and durable material such as rawhide, ropes made from natural or synthetic fibers, and polymeric articles made from nylon, polyester or thermoplastic polyurethane. As the animal chews, licks or gnaws the product, the incorporated active elements are released into the animal's oral cavity into a salivary medium, comparable to an effective brushing or rinsing.

EXAMPLES

The following examples further describe and demonstrate embodiments within the scope of the present invention. These examples are given solely for the purpose of illustration and are not to be construed as limitations of the present invention.

Example 1. Mouth Rinse Compositions

Mouth rinse emulsion compositions A - F according to the present invention are shown below with amounts of components in weight %.

| Components | A | B | C | D | E | F |
|---|---|---|---|---|---|---|
| Water | QS | QS | QS | QS | QS | QS |

(continued)

| Components | A | B | C | D | E | F |
|---|---|---|---|---|---|---|
| Glycerin | 5 | 5 | 5 | 5 | 7.5 | 10 |
| Propylene glycol | - | 2 | - | 3 | - | - |
| Ethanol | - | - | 5 | - | 3 | 10 |
| Methyl Paraben | - | 0.02 | 0.02 | - | - | - |
| Propyl Paraben | - | 0.005 | 0.005 | - | - | - |
| CPC | 0.03 | 0.07 | 0.1 | 0.05 | 0.07 | 0.1 |
| Sucralose | 0.03 | 0.04 | 0.06 | 0.05 | 0.05 | 0.07 |
| Anisaldehvde | | | | 0.1 | - | 0.1 |
| CM Dextran | | | | 0.05 | 0.05 | 0.1 |
| Flavor/sensate oils | 0.05 | 0.2 | 0.1 | 0.3 | 0.3 | 0.4 |
| Performathox 490 | 0.075 | - | 0.05 | 0.1 | 0.05 | 0.05 |

Example 2. *In Vitro* Biofilm Reduction

The effects of the present mouth rinse emulsions with high flavor oil loading were compared to traditional clear rinses using an *in vitro* Particle Based Biofilm (PBB) model described below. The samples tested are shown below in TABLE 1 and the results of the testing in Tables 2 and 3.

TABLE 1

| Samples | Sample Descriptions |
|---|---|
| Saline | Sterile 0.9% saline (null control) |
| Emulsion base | Emulsion base comprised of 2.8% ethanol, 7.5% glycerin, 0.075% sucralose, 89.625% USP water |
| ProHealth | Crest Pro Health clear mouth rinse comprised of 0.12% flavor oil and 0.07%ppm CPC; lot #00615395UA, Expiration 01/2012 |
| Emulsion Lo | Low particle size [115 d.nm] emulsion rinse comprised of 99.6% emulsion base, 0.3% oil, 0.1% CPC |
| Emulsion Md | Mid particle size [828 d.nm] emulsion rinse comprised of 99.6% emulsion base, 0.3% oil, 0.1% CPC |
| Emulsion Hi | High particle size [5,850 d.nm] emulsion rinse comprised of 99.6% emulsion base, 0.3% oil, 0.1% CPC |

Human saliva was collected daily from five to seven donors to culture PBBs. Saliva donors were required to meet minimum selection criteria, including but not limited to:

- Between the ages of 18 and 50 years
- No prophylaxis within the past 4 weeks nor undergoing treatment for any oral nor dental disease
- No use of any type of mouth rinse, floss or toothpick within last 48h or during collection period
- No use of antibacterial toothpaste of any kind within last 48h or during collection period
- No fever (>38°C or 100°F) and/or communicable disease or oral infection within past 48h or during collection period
- No use of antihistamines, decongestants or other cold/flu/allergy medicines within past 48h or during collection period
- No use of oral antibiotics within the past 7 days or during collection
- No use of tobacco products of any kind
- Females may not be pregnant nor lactating nor taking oral steroid medications within the past 7 days or during collection

At least 48h prior to commencing and during saliva collection donors were required to practice the following oral hygiene, including, but not limited to:

- Brush teeth with a supplied Cavity Protection dentifrice containing sodium fluoride and a supplied standard manual toothbrush no more than twice daily during a 'washout' period beginning at least 48h prior to first collection day and continuing through the saliva collection period
- No use of antibacterial toothpaste, mouth rinses, floss or toothpicks during the washout and saliva collection periods

Saliva was collected on three consecutive mornings. For each morning saliva collection was done after donors awoke and prior to eating, drinking or performing oral hygiene. To stimulate saliva flow donors chewed on a supplied sterile piece of paraffin with beeswax or polypropylene tubing. Donors either warmed the paraffin chew for 10 to 20sec in a microwave or by placing between the cheek and gum for at least 1min prior to chewing to reduce flaking of the chew. Donors periodically spit their saliva directly into a sterile 100mL wide-mouth collection container until at least 25mL of saliva was collected. The collection container was sealed with its lid and the container placed on ice for transport to the appropriate microbiology lab. The used paraffin or tubing is discarded after each day's use.

Saliva was prepared for use as follows. Donor saliva was kept on ice or refrigerated until all samples were obtained in the microbiology laboratory. Equal amounts, typically 20mL, of saliva from each of at least five donors was pooled into a sterile Erlenmeyer ($\geq$500mL volume) with 10 or more sterile glass beads of 5 to 6mm diameter. Any settled solid material in the saliva containers was avoided in the transferred saliva. The pooled saliva was vortexed at high speed, at least 200rpm, sufficient to circulate the glass beads through the pooled saliva for at least 60sec to homogenize the saliva and break up any viscous globules. The product of the this procedure was sheared pooled saliva (SPS). SPS was diluted with an equal quantity of sterile 0.9% saline to form sheared pooled saliva diluted (SPSD). For the first day of culturing PBBs SPSD was amended to contain 1% sucrose.

For culturing PBB's, 20 ml SPSD with 1% sucrose was added to eight 50ml centrifuge tubes containing 725 to 775 mg sterile hydroxyapatite powder (HAP). The HAP had a 53 to 124$\mu$m mean diameter and was obtained from Clarkson Chromatography Products Inc., South Williamsport, PA USA 17702. The HAP was first sterilized by spreading 10 to 40 g of the powder in an open 100mm glass petri dish. The petri dish with HAP was transferred into a biosafety cabinet and sterilized under an ultraviolet (UV) light source. A sterile cell spreader was used to redistribute the HAP every 1 to 3 h during the work day with a minimum 48h continuous UV exposure prior to use.

The 50ml centrifuge tubes were sealed with their caps and transferred to a 31 to 35 °C aerobic incubator. The 50ml centrifuge tubes were laid on their sides on a Rocking Platform Model 200 (VWR Scientific Products, Radnor, PA) and held in place with bungee cords. The rocking table was set to a speed sufficient to maintain a majority of the particles in constant motion -moving from the bottom of the 50ml centrifuge tubes to the capped end with every rock of the platform.

After 22 to 24h the 50ml centrifuge tubes were removed from the incubator and the SPSD culture medium replaced as follows: The 50ml centrifuge tubes were allowed to stand upright at ambient laboratory temperature for at least 1min to settle the bulk of PBBs to the bottom of the 50ml centrifuge tubes. All the SPSD supernatant except for about 1ml from each 50ml centrifuge tube was aspirated with a sterile pipet and discarded. To each 50ml centrifuge tube was added 20ml fresh SPSD collected that morning without any sucrose amendment. The 50ml centrifuge tubes were sealed with their caps and placed as before in the incubator on the rocker table to incubate. After an additional 22 to 24h incubation the culture medium was renewed in the same manner used after the first incubation period and the PBB 50ml centrifuge tubes returned to the incubator for an additional 22 to 24h incubation on the rocker table.

After 68 to 70h total incubation the 50ml centrifuge tubes were removed from the incubator. The 50ml centrifuge tubes were allowed to stand upright at ambient laboratory temperature for at least 1min to settle the bulk of PBBs to the bottom of the 50ml centrifuge tubes. All the SPSD supernatant except for about 1ml from each 50ml centrifuge tube was aspirated with a sterile pipet and discarded. To each 50ml centrifuge tube was added 20ml sterile saline and the chamber caps replaced. Each chamber was inverted five times about once per second to effect the first washing of PBBs. The 50ml centrifuge tubes were allowed to stand upright at ambient laboratory temperature for at least 1min to settle the bulk of PBBs to the bottom of the 50ml centrifuge tubes. All the saline wash supernatant except for about 1ml from each 50ml centrifuge tube was aspirated with a sterile pipet and discarded. A second saline wash was performed by repeating saline addition, chamber inversions and supernatant aspiration. After two saline washes to each 50ml centrifuge tube was added 10ml sterile saline to resuspend the washed PBBs.

After the washing and resuspension of PBBs all of the PBBs plus resuspension medium were transferred to a 100ml Eppendorf reservoir for use in an epMotion 5075 automated pipetting system (Eppendorf, Hamburg, Germany). Proprietary programs for the epMotion 5075 were executed to perform the PBB method to assay for biofilm reduction after a single 1min treatment.

The PBB method began with the transfer of four random 50$\mu$l sample of washed PBBs to all wells of a sterile 96-deepwell (2.2ml well capacity) dose plate. An additional 400$\mu$l of reservoir medium supernatant was transferred to each well of the dose plate. To expose all PBBs to the dosing solution, 1 ml of saline control or other treatment sample was added to each of six dose plate wells with mixing. After 10 to 15 sec 1 ml of supernatant was removed from dosed wells and discarded. One ml of Dey Engley Neutralizing Broth (DEB) was added with mixing to all the dosed wells 55 to 65 sec after the dosing solution(s) were first added to the dose plate. DEB was added to mitigate any further bacteriocidal or bacteriostatic activity by anionic, cationic and nonionic substances remaining in the dose plate wells from the dosing solution(s) and to provide nutrients to the surviving bacteria to enable their recovery from the dosing. After a 30 to 45min recovery period 1ml of supernatant from dose plate wells was removed and 1ml sterile saline added with mixing to wash the dosed and neutralized PBBs. After 10 to 15 sec 1 ml of supernatant was removed from the dose plate wells and discarded. Another 1ml sterile saline added with mixing to wash the dosed and neutralized PBBs a second time. After 10 to 15 sec 1 ml of supernatant was removed from the dose plate wells and discarded. Dosed, neutralized and washed (DNR) PBBs were transferred as two random 50 $\mu$l samples from each dose plate well into its respective well in a sterile opaque 96-well assay plate.

BacTiter-Glo® (BTG) (Promega Corporation, Madison, WI) was used to determine bacterial ATP (adenosine triphosphate) in the assay plate wells containing DNR PBB (intact biofilm). ATP is a measure of biofilm functional state; specifically, bacterial metabolic energy. Lower intact biofilm ATP means biofilm bacteria were killed and/or their metabolism reduced and/or the biofilm was dispersed (without necessarily killing bacteria). To all wells in the assay plate was added 95$\mu$l BTG. The ATP assay plate was incubated at ambient room temperature, 20 to 22°C, for 10 min on an orbital shaker table at 750 rpm in the dark. After incubation the assay was transferred to a plate reader and luminescence read for each well as relative luminescence units (RLU).

The ATP (RLU) results for intact biofilm were Log10-transformed prior to charting and statistical analysis. The sample mean log-transformed data with 95% confidence limits (CL) are plotted below in TABLE 3. The sample log means and standard errors (SE) are shown below in TABLE 2, as well as the percent biofilm reduction from the saline control for each sample. Log-transformed data were also statistically analyzed using JMP version 9.01 (SAS Corporation) by ANOVA and all means comparisons made using Tukey HSD.

As shown in TABLE 2 and TABLE 3, a marketed high-performance clear rinse, Crest Pro Health, reduced biofilm ATP significantly ($p<0.05$) more than the saline control. In relative terms, PBBs treated with Crest Pro Health had about 34% less ATP activity versus PBBs treated with saline. This is roughly equivalent to the relative oral biofilm reductions *in vivo* after rinsing with Crest Pro Health versus water. Importantly, all the present emulsion forms reduced biofilm ATP significantly ($p<0.05$) more than Crest Pro Health. In relative terms, the prototype emulsion rinses doubled the Crest Pro Health reduction in biofilm ATP activity versus saline. Moreover, the Emulsion Lo sample with smallest oil droplet particle sizes reduced biofilm ATP significantly ($p<0.05$) more than the emulsions with larger oil droplet particle sizes.

TABLE 2

| Sample | # of Samples | Intact Biofilm Mean Log10 RLU ± SE | Mean Percent Biofilm Reduction versus Saline |
|---|---|---|---|
| Saline | 12 | 5.697 ± 0.014 (A) | - |
| Emulsion base | 6 | 5.725 ± 0.012 (A) | NA |
| ProHealth | 6 | 5.515 ± 0.019 (B) | 34% |
| Emulsion Lo | 6 | 5.123 ± 0.016 (D) | 73% |
| Emulsion Md | 6 | 5.211 ± 0.030 (C) | 67% |
| Emulsion Hi | 6 | 5.239 ± 0.011 (C) | 65% |
| Log10 ATP means with different letters are significantly different at $p<0.05$ level based on Tukey HSD all means comparison. | | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the scope of the invention. The scope of the claims should not be limited by the embodiments set forth in the examples, but should be given the broadest interpretation consistent with the description as a whole.

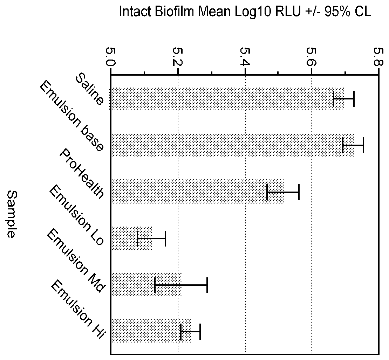

**Claims**

1.  An oral care mouth rinse compositions comprising:

    (a) from 0.025 % to 0.1 % by weight of cetylpyridinium chloride as antimicrobial agent,
    (b) at least 0.05 % by weight of an essentially water-insoluble volatile flavor oil,
    (c) an orally-acceptable carrier comprising water at a level of least 50 % by weight of the composition, and
    (d) less than 0.1% total additional surfactant by weight of the composition,

    wherein the composition is a stable oil-in-water nano-emulsion having a dispersed phase comprising oil droplets having an average mean particle size of 30 to 350 nm, wherein the nano-emulsion does not form spontaneously, but requires energy input for formation, and are not thermodynamically stable once formed.

2.  An oral care mouth rinse composition according to Claim 1, comprising from 0.05% to 5% by weight of the essentially water-insoluble volatile flavor oil.

3.  An oral care mouth rinse composition according to any of Claims 1 to 2, comprising from 50% to 95% water.

4.  An oral care mouth rinse composition according to any of Claims 1 to 3, comprising from 0 to 10 % by weight ethanol.

5.  An oral care mouth rinse composition according to any of Claims 1 to 4, wherein the oil droplets have an average mean particle size of 30 to 200 nm.

6.  An oral care mouth rinse composition according to any of Claims 1 to 5 further comprising one or more carrier materials selected from a fluoride ion source, additional antimicrobial agent, an anti-inflammatory agent, an anticalculus agent, a desensitizing agent, a peroxide source, a tooth substantive agent, a surfactant, an emulsifying agent, an anti-stain agent, humectants, essential oils, a coolant, a sweetening agent or other sensates.

7.  An oral care mouth rinse composition according to Claim 6, wherein the additional antimicrobial agent comprises one or a mixture of metal ion sources to provide stannous, zinc or copper ions.

8.  An oral care mouth rinse composition according to Claim 7, wherein the metal ion sources comprise one or a mixture of stannous fluoride, stannous chloride, stannous chloride dihydrate, zinc citrate, zinc lactate, zinc sulfate, zinc chloride, zinc acetate, zinc oxide, copper sulfate, and copper gluconate.

9.  An oral care mouth rinse composition according to any of Claims 1 to 8, having a bioavailability of the quaternary ammonium agent of at least 50% as measured using an *in vitro* Disk Retention Assay (DRA).

10. An oral care mouth rinse composition according to any of Claims 1 to 9, having a reduction in biofilm adenosine triphosphate (ATP) activity of at least 30% as measured using an *in vitro* Particle Based Biofilm (PBB) assay.

11. Composition for use in a method for controlling dental plaque and calculus in a subject in need thereof, comprising administering to the oral cavity of the subject an oral care mouth rinse compositions of any one of claims 1 to 10.

**Patentansprüche**

1.  Mundpflege-Mundspülungszusammensetzungen, umfassend:

    (a) von 0,025 Gew.-% bis 0,1 Gew.-% Cetylpyridiniumchlorid als antimikrobiellen Wirkstoff,
    (b) wenigstens 0,05 Gew.-% eines im Wesentlichen wasserunlöslichen, flüchtigen Aromaöls,
    (c) einen oral akzeptablen Träger, umfassend Wasser in einer Menge von wenigstens 50 Gew.-% der Zusammensetzung, und
    (d) weniger als 0,1 Gew.-% der Zusammensetzung insgesamt eines zusätzlichen Tensids,

wobei die Zusammensetzung eine stabile Öl-in-Wasser-Nanoemulsion ist, die eine disperse Phase aufweist, die Öltröpfchen umfasst, die eine durchschnittliche mittlere Teilchengröße von 30 bis 350 nm aufweisen, wobei sich die Nanoemulsion nicht spontan bildet, sondern Energiezufuhr zur Bildung benötigt, und nach dem Bilden nicht thermodynamisch stabil ist.

2. Mundpflege-Mundspülungszusammensetzung nach Anspruch 1, umfassend von 0,05 Gew.-% bis 5 Gew.-% des im Wesentlichen wasserunlöslichen, flüchtigen Aromaöls.

3. Mundpflege-Mundspülungszusammensetzung nach einem der Ansprüche 1 bis 2, umfassend von 50 % bis 95 % Wasser.

4. Mundpflege-Mundspülungszusammensetzung nach einem der Ansprüche 1 bis 3, umfassend von 0 bis 10 Gew.-% Ethanol.

5. Mundpflege-Mundspülungszusammensetzung nach einem der Ansprüche 1 bis 4, wobei die Öltröpfchen eine durchschnittliche mittlere Teilchengröße von 30 bis 200 nm aufweisen.

6. Mundpflege-Mundspülungszusammensetzung nach einem der Ansprüche 1 bis 5, ferner umfassend einen oder mehrere Trägerstoffe, ausgewählt aus einer Fluoridionenquelle, zusätzlichem antimikrobiellen Wirkstoff, einem entzündungshemmenden Mittel, einem Antizahnsteinmittel, einem Desensibilisierungsmittel, einer Peroxidquelle, einem Zahnsubstanzmittel, einem Tensid, einem Emulgator, einem Antiverfärbungsmittel, Feuchthaltemitteln, ätherischen Ölen, einem Kühlmittel, einem Süßstoff oder anderem sinnlich Wahrgenommenem.

7. Mundpflege-Mundspülungszusammensetzung nach Anspruch 6, wobei der zusätzliche antimikrobielle Wirkstoff eine oder eine Mischung aus Metallionenquellen umfasst, um Zinn-, Zink- oder Kupferionen bereitzustellen.

8. Mundpflege-Mundspülungszusammensetzung nach Anspruch 7, wobei die Metallionenquellen eins oder eine Mischung aus Zinnfluorid, Zinn(II)-chlorid, Zinnchlorid-Dihydrat, Zinkcitrat, Zinklactat, Zinksulfat, Zinkchlorid, Zinkacetat, Zinkoxid, Kupfersulfat und Kupfergluconat umfassen.

9. Mundpflege-Mundspülungszusammensetzung nach einem der Ansprüche 1 bis 8, die eine Bioverfügbarkeit der quartären Ammoniumverbindung von wenigstens etwa 50 % bei Messung in einem *In-vitro*-Disk-Retention-Assay (DRA) aufweist.

10. Mundpflege-Mundspülungszusammensetzung nach einem der Ansprüche 1 bis 9, die eine Reduktion der Biofilm-Adenosintriphosphat-Aktivität (ATP-Aktivität) von wenigstens 30 % bei Messung mithilfe eines *In-vitro*-Particle-Based-Biofilm-Assays (PBB-Assays) aufweist.

11. Zusammensetzung zum Gebrauch in einem Verfahren zum Eindämmen von Zahnbelag und Zahnstein bei einem Individuum, das dies benötigt, umfassend Verabreichen einer Mundpflege-Mundspülungszusammensetzungen nach einem der Ansprüche 1 bis 10 an die Mundhöhle des Individuums.

**Revendications**

1. Compositions de rinçage de bouche pour soins bucco-dentaires, comprenant :

(a) de 0,025 % à 0,1 % en poids de chlorure de cétylpyridinium en tant qu'agent antimicrobien,
(b) au moins 0,05 % en poids d'une huile aromatisante volatile sensiblement insoluble dans l'eau,
(c) un véhicule oralement acceptable comprenant de l'eau à un taux de moins de 50 % en poids de la composition, et
(d) moins de 0,1 % d'agent tensioactif supplémentaire total en poids de la composition,

dans lesquelles la composition est une nano-émulsion huile-dans-eau stable ayant une phase dispersée comprenant des gouttelettes d'huile ayant une taille de particules moyenne arithmétique de 30 à 350 nm, dans lesquelles la nano-émulsion ne se forme pas spontanément, mais exige un apport énergétique pour la formation, et elles ne sont pas thermodynamiquement stables une fois formées.

**2.** Composition de rinçage de bouche pour soins bucco-dentaires selon la revendication 1, comprenant de 0,05 % à 5 % en poids de l'huile aromatisante volatile sensiblement insoluble dans l'eau.

**3.** Composition de rinçage de bouche pour soins bucco-dentaires selon l'une quelconque des revendications 1 à 2, comprenant de 50 % à 95 % d'eau.

**4.** Composition de rinçage de bouche pour soins bucco-dentaires selon l'une quelconque des revendications 1 à 3, comprenant de 0 % à 10 % en poids d'éthanol.

**5.** Composition de rinçage de bouche pour soins bucco-dentaires selon l'une quelconque des revendications 1 à 4, dans laquelle les gouttelettes d'huile ont une taille de particules moyenne arithmétique de 30 à 200 nm.

**6.** Composition de rinçage de bouche pour soins bucco-dentaires selon l'une quelconque des revendications 1 à 5, comprenant en outre un ou plusieurs matériaux formant véhicule, choisis parmi une source d'ions fluorure, un agent antimicrobien supplémentaire, un agent anti-inflammatoire, un agent antitartre, un agent désensibilisant, une source de peroxyde, un agent substantif sur les dents, un agent tensioactif, un agent émulsifiant, un agent anti-coloration, des humectants, des huiles essentielles, un agent refroidissant, un agent édulcorant ou d'autres sensates.

**7.** Composition de rinçage de bouche pour soins bucco-dentaires selon la revendication 6, dans laquelle l'agent antimicrobien supplémentaire comprend une source d'ions métalliques ou un mélange de sources d'ions métalliques pour fournir des ions stanneux, zinc ou cuivre.

**8.** Composition de rinçage de bouche pour soins bucco-dentaires selon la revendication 7, dans laquelle les sources d'ions métalliques comprennent un parmi, ou un mélange de, fluorure stanneux, chlorure stanneux, chlorure stanneux dihydraté, citrate de zinc, lactate de zinc, sulfate de zinc, chlorure de zinc, acétate de zinc, oxyde de zinc, sulfate de cuivre et gluconate de cuivre.

**9.** Composition de rinçage de bouche pour soins bucco-dentaires selon l'une quelconque des revendications 1 à 8, ayant une biodisponibilité de l'agent ammonium quaternaire d'au moins environ 50 %, telle que mesurée en utilisant un essai de rétention de disque (DRA) *in vitro.*

**10.** Composition de rinçage de bouche pour soins bucco-dentaires selon l'une quelconque des revendications 1 à 9, ayant une réduction d'activité d'adénosine-triphosphate (ATP) de biofilm d'au moins 30 % telle que mesurée en utilisant un essai de biofilm à base de particules (PBB) *in vitro.*

**11.** Composition pour utilisation dans un procédé de lutte contre la plaque et le tartre dentaires chez un sujet qui en a besoin, comprenant l'administration à la cavité buccale du sujet de compositions de rinçage de bouche pour soins bucco-dentaires selon l'une quelconque des revendications 1 à 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012076309 A1
- WO 2012076295 A1
- WO 2008005705 A1
- US 4206215 A, Bailey
- WO 05072693 A
- US 5004597 A, Majeti
- US 5578293 A, Prencipe
- US 5281410 A, Lukacovic
- US 4022880 A
- US 5534243 A
- US 20080253976 A1
- US 3535421 A, Briner
- US 5180577 A, Polefka
- US 3988433 A, Benedict
- US 5213790 A
- US 5145666 A
- US 4849213 A
- US 4528180 A, Schaeffer
- US 4083955 A, Grabenstetter
- US 5198220 A
- US 5242910 A, Damani
- WO 2008057136 A1, Procter & Gamble, Doyle
- US 4627977 A, Gaffar
- US 3678154 A, Widder
- US 3070510 A, Cooley and Grabenstetter
- US 3538230 A, Pader
- US 3862307 A, DiGiulio
- US 4340583 A, Wason
- US 5603920 A
- US 5589160 A
- US 5658553 A
- US 5651958 A
- US 6740311 B
- US 5292501 A
- US 5213789 A
- US 5093170 A
- US 5009882 A
- US 4939284 A
- US 5011913 A, Benedict
- US 4877603 A, Degenhardt
- US 4749758 A, Dursch
- GB 1290724 A
- US 5980776 A, Zakikhani
- US 6071434 A, Davis
- US 4138477 A
- US 4183914 A, Gaffar
- US 3492131 A
- US 4459425 A, Amano
- US 4136163 A
- US 4150052 A
- US 4153679 A
- US 4157384 A
- US 4178459 A
- US 4230688 A
- WO 2005049553 A1

### Non-patent literature cited in the description

- **KIRK-OTHMER.** Encyclopedia of Chemical Technology
- The Merck Index
- *Am J. Dent.,* 2005, vol. 18, 15A-17A
- **S. J. HUNTER-RINDERLE et al.** Evaluation of Cetylpyridinium Chloride-Containing Mouthwashes Using In Vitro Disk Retention and Ex Vivo Plaque Glycolysis Methods. *J. Clin. Den.,* 1997, vol. 8, 107-113
- Oral Health Care Drug Products For Over-The-Counter Human Use; Antigingivitis/Antiplaque Drug Products; Establishment of a Monograph: Proposed Rules. *Federal Register,* vol. 68 (103
- Dairy Products. Official Methods of Analysis of the Association of Chemical Analytical Chemists. 1980, vol. 16, 256
- Kirk-Othmer Encyclopedia of Chemical Technology. Wiley-Interscience, 1982, vol. 17